(19) Europäisches Patentamt
European Patent Office
Office européen des brevets

(11) EP 2 471 929 A1

(12) **EUROPEAN PATENT APPLICATION**

(43) Date of publication:
**04.07.2012 Bulletin 2012/27**

(21) Application number: **10016162.9**

(22) Date of filing: **29.12.2010**

(51) Int Cl.:
***C12N 15/82*** *(2006.01)* ***C12P 21/00*** *(2006.01)*
*C12R 1/89* *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Algenics**
**44800 Saint Herblain (FR)**

(72) Inventors:
- **Carlier, Aude**
  **44000 Nantes (FR)**
- **Michel, Rémy**
  **44100 Nantes (FR)**
- **Dufourmantel, Nathalie**
  **44130 Fay De Bretagne (FR)**
- **Cadoret, Jean-Paul**
  **44115 Basse Goulaine (FR)**
- **Lejeune, Alexandre**
  **44240 La Chapelle Sur Erdre (FR)**

(74) Representative: **Barbot, Willy**
**SIMODORO**
**Parc Cézanne II, Bât. G**
**290 Avenue Galilée**
**13857 Aix en Provence Cedex 3 (FR)**

(54) **Production of high mannose glycosylated proteins stored in the plastid of microalgae**

(57) The present invention concerns a transformed microalga producing a protein harboring a non-immunogenic "high mannose" pattern of glycosylation in the plastid of said transformed microalga, wherein 1) said transformed microalga has a Chloroplast Endoplasmic Reticulum (CER); 2) said microalga has been transformed with a nucleic acid sequence operatively linked to a promoter, said nucleic acid sequence encoding an amino acid sequence comprising (i) an amino-terminal bipartite topogenic signal (BTS) sequence composed of at least a signal peptide followed by a transit peptide ; and (ii) The sequence of said protein,3) the xylosyltransferases and fucosyltransferases of said microalga have not been inactivated; 4) the N-acetylglycosyltransferase I of said microalga has not been inactivated, preferably the N-acetylglycosyltranferases II, III, IV, V and VI, mannosidase II and glycosyltransferases of said microalga have not been inactivated

EP 2 471 929 A1

SEQUENCE LISTING

<110> ALGENICS
CARLIER, Aude
MICHEL, Rémy
DUFOURMANTEL, Nathalie
CADORET, Jean-Paul
LEJEUNE , Alexandre

<120> PRODUCTION OF HIGH MANNOSE GLYCOSYLATED PROTEINS STORED IN THE PLASTID OF MICROALGAE

<130> ALG-B-0002EP1

<160> 43

<170> PatentIn version 3.5

<210> 1
<211> 46
<212> PRT
<213> Guillardia theta

<400> 1

```
Met Ile Arg Ala Cys Ala Leu Leu Gly Leu Ala Ala Ser Ala Ala Ala
1               5                   10                  15

Phe Ala Pro Ser Ser Leu Pro Ile Arg Ala Asn Arg Ala Ser Ala Val
            20                  25                  30

Ser Lys Met Ser Met Gln Ser Asn Arg Phe Ser Tyr Arg Ser
        35                  40                  45
```

<210> 2
<211> 55
<212> PRT
<213> Phaeodactylum tricornutum

<400> 2

```
Met Arg Ser Phe Cys Ile Ala Ala Leu Leu Ala Val Ala Ser Ala Phe
1               5                   10                  15

Thr Thr Gln Pro Thr Ser Phe Thr Val Lys Thr Ala Asn Val Gly Glu
            20                  25                  30

Arg Ala Ser Gly Val Phe Pro Glu Gln Ser Ser Ala His Arg Thr Arg
        35                  40                  45

Lys Ala Thr Ile Val Met Asp
    50                  55
```

<210> 3
<211> 54
<212> PRT
<213> Phaeodactylum tricornutum

<400> 3

```
Met Lys Val Ala Thr Thr Leu Thr Leu Ala Phe Ile Cys Cys Ala Ser
```

1 5 10 15

Ala Phe Gly Leu Asn Gly Gln Thr Thr Ser Val Met Lys Lys Val Gly
20 25 30

Phe Asp Ala Gly Ser Lys Pro Met Val Gln Ala Ile Asp Val Gln Gly
35 40 45

Asn Arg Leu Gly Ser Asn
50

<210> 4
<211> 30
<212> PRT
<213> Phaeodactylum tricornutum

<400> 4

Met Lys Thr Ala Val Ile Ala Ser Leu Ile Ala Gly Ala Ala Ala Phe
1 5 10 15

Ala Pro Ala Lys Asn Ala Ala Arg Thr Ser Val Ala Thr Asn
20 25 30

<210> 5
<211> 104
<212> PRT
<213> Phaeodactylum tricornutum

<400> 5

Met Gly Arg Gly Val Ile Ile Phe Cys Val Lys Asn Phe Ala Val Trp
1 5 10 15

Leu Leu Ile Ile Thr Ser Ala Val Ser Ile Gln Ala Trp Ile Pro Leu
20 25 30

Pro Leu Ser Ala Thr Val Lys Ala Arg Ile Asp Ser Thr Thr Leu Phe
35 40 45

Phe Ser Arg Tyr Lys Thr Pro Leu Tyr His Gly Gly Asn Glu Glu Ser
50 55 60

Tyr Gly Pro Pro Ala Pro Ala Val Asp Ser Arg Tyr Tyr Thr Tyr Val
65 70 75 80

Glu Ala Pro Val Gln Ser Ser Arg Ser Arg Asp Thr Lys Gln Pro Ile
85 90 95

Thr Leu Ser Arg Phe Leu Ser Asp
100

<210> 6
<211> 43
<212> PRT

<213> Phaeodactylum tricornutum

<400> 6

Met Lys Phe Thr Ala Ala Cys Ser Ile Ala Leu Ala Ala Ser Ala Ser
1               5                   10                  15

Ala Phe Ala Pro Ile Pro Ser Val Ser Arg Thr Thr Asp Leu Ser Met
            20                  25                  30

Ser Leu Gln Lys Asp Leu Ala Asn Val Gly Lys
        35                  40

<210> 7
<211> 497
<212> PRT
<213> Homo sapiens

<400> 7

Ala Arg Pro Cys Ile Pro Lys Ser Phe Gly Tyr Ser Ser Val Val Cys
1               5                   10                  15

Val Cys Asn Ala Thr Tyr Cys Asp Ser Phe Asp Pro Pro Thr Phe Pro
            20                  25                  30

Ala Leu Gly Thr Phe Ser Arg Tyr Glu Ser Thr Arg Ser Gly Arg Arg
        35                  40                  45

Met Glu Leu Ser Met Gly Pro Ile Gln Ala Asn His Thr Gly Thr Gly
    50                  55                  60

Leu Leu Leu Thr Leu Gln Pro Glu Gln Lys Phe Gln Lys Val Lys Gly
65                  70                  75                  80

Phe Gly Gly Ala Met Thr Asp Ala Ala Ala Leu Asn Ile Leu Ala Leu
                85                  90                  95

Ser Pro Pro Ala Gln Asn Leu Leu Leu Lys Ser Tyr Phe Ser Glu Glu
            100                 105                 110

Gly Ile Gly Tyr Asn Ile Ile Arg Val Pro Met Ala Ser Cys Asp Phe
        115                 120                 125

Ser Ile Arg Thr Tyr Thr Tyr Ala Asp Thr Pro Asp Asp Phe Gln Leu
    130                 135                 140

His Asn Phe Ser Leu Pro Glu Glu Asp Thr Lys Leu Lys Ile Pro Leu
145                 150                 155                 160

Ile His Arg Ala Leu Gln Leu Ala Gln Arg Pro Val Ser Leu Leu Ala
                165                 170                 175

Ser Pro Trp Thr Ser Pro Thr Trp Leu Lys Thr Asn Gly Ala Val Asn

180 185 190

Gly Lys Gly Ser Leu Lys Gly Gln Pro Gly Asp Ile Tyr His Gln Thr
195 200 205

Trp Ala Arg Tyr Phe Val Lys Phe Leu Asp Ala Tyr Ala Glu His Lys
210 215 220

Leu Gln Phe Trp Ala Val Thr Ala Glu Asn Glu Pro Ser Ala Gly Leu
225 230 235 240

Leu Ser Gly Tyr Pro Phe Gln Cys Leu Gly Phe Thr Pro Glu His Gln
245 250 255

Arg Asp Phe Ile Ala Arg Asp Leu Gly Pro Thr Leu Ala Asn Ser Thr
260 265 270

His His Asn Val Arg Leu Leu Met Leu Asp Asp Gln Arg Leu Leu Leu
275 280 285

Pro His Trp Ala Lys Val Val Leu Thr Asp Pro Glu Ala Ala Lys Tyr
290 295 300

Val His Gly Ile Ala Val His Trp Tyr Leu Asp Phe Leu Ala Pro Ala
305 310 315 320

Lys Ala Thr Leu Gly Glu Thr His Arg Leu Phe Pro Asn Thr Met Leu
325 330 335

Phe Ala Ser Glu Ala Cys Val Gly Ser Lys Phe Trp Glu Gln Ser Val
340 345 350

Arg Leu Gly Ser Trp Asp Arg Gly Met Gln Tyr Ser His Ser Ile Ile
355 360 365

Thr Asn Leu Leu Tyr His Val Val Gly Trp Thr Asp Trp Asn Leu Ala
370 375 380

Leu Asn Pro Glu Gly Gly Pro Asn Trp Val Arg Asn Phe Val Asp Ser
385 390 395 400

Pro Ile Ile Val Asp Ile Thr Lys Asp Thr Phe Tyr Lys Gln Pro Met
405 410 415

Phe Tyr His Leu Gly His Phe Ser Lys Phe Ile Pro Glu Gly Ser Gln
420 425 430

Arg Val Gly Leu Val Ala Ser Gln Lys Asn Asp Leu Asp Ala Val Ala
435 440 445

Leu Met His Pro Asp Gly Ser Ala Val Val Val Val Leu Asn Arg Ser

450 455 460

Ser Lys Asp Val Pro Leu Thr Ile Lys Asp Pro Ala Val Gly Phe Leu
465 470 475 480

Glu Thr Ile Ser Pro Gly Tyr Ser Ile His Thr Tyr Leu Trp His Arg
485 490 495

Gln

<210> 8
<211> 398
<212> PRT
<213> Homo sapiens

<400> 8

Leu Asp Asn Gly Leu Ala Arg Thr Pro Thr Met Gly Trp Leu His Trp
1 5 10 15

Glu Arg Phe Met Cys Asn Leu Asp Cys Gln Glu Glu Pro Asp Ser Cys
20 25 30

Ile Ser Glu Lys Leu Phe Met Glu Met Ala Glu Leu Met Val Ser Glu
35 40 45

Gly Trp Lys Asp Ala Gly Tyr Glu Tyr Leu Cys Ile Asp Asp Cys Trp
50 55 60

Met Ala Pro Gln Arg Asp Ser Glu Gly Arg Leu Gln Ala Asp Pro Gln
65 70 75 80

Arg Phe Pro His Gly Ile Arg Gln Leu Ala Asn Tyr Val His Ser Lys
85 90 95

Gly Leu Lys Leu Gly Ile Tyr Ala Asp Val Gly Asn Lys Thr Cys Ala
100 105 110

Gly Phe Pro Gly Ser Phe Gly Tyr Tyr Asp Ile Asp Ala Gln Thr Phe
115 120 125

Ala Asp Trp Gly Val Asp Leu Leu Lys Phe Asp Gly Cys Tyr Cys Asp
130 135 140

Ser Leu Glu Asn Leu Ala Asp Gly Tyr Lys His Met Ser Leu Ala Leu
145 150 155 160

Asn Arg Thr Gly Arg Ser Ile Val Tyr Ser Cys Glu Trp Pro Leu Tyr
165 170 175

Met Trp Pro Phe Gln Lys Pro Asn Tyr Thr Glu Ile Arg Gln Tyr Cys
180 185 190

Asn His Trp Arg Asn Phe Ala Asp Ile Asp Asp Ser Trp Lys Ser Ile
195 200 205

Lys Ser Ile Leu Asp Trp Thr Ser Phe Asn Gln Glu Arg Ile Val Asp
210 215 220

Val Ala Gly Pro Gly Gly Trp Asn Asp Pro Asp Met Leu Val Ile Gly
225 230 235 240

Asn Phe Gly Leu Ser Trp Asn Gln Gln Val Thr Gln Met Ala Leu Trp
245 250 255

Ala Ile Met Ala Ala Pro Leu Phe Met Ser Asn Asp Leu Arg His Ile
260 265 270

Ser Pro Gln Ala Lys Ala Leu Leu Gln Asp Lys Asp Val Ile Ala Ile
275 280 285

Asn Gln Asp Pro Leu Gly Lys Gln Gly Tyr Gln Leu Arg Gln Gly Asp
290 295 300

Asn Phe Glu Val Trp Glu Arg Pro Leu Ser Gly Leu Ala Trp Ala Val
305 310 315 320

Ala Met Ile Asn Arg Gln Glu Ile Gly Gly Pro Arg Ser Tyr Thr Ile
325 330 335

Ala Val Ala Ser Leu Gly Lys Gly Val Ala Cys Asn Pro Ala Cys Phe
340 345 350

Ile Thr Gln Leu Leu Pro Val Lys Arg Lys Leu Gly Phe Tyr Glu Trp
355 360 365

Thr Ser Arg Leu Arg Ser His Ile Asn Pro Thr Gly Thr Val Leu Leu
370 375 380

Gln Leu Glu Asn Thr Met Gln Met Ser Leu Lys Asp Leu Leu
385 390 395

<210> 9
<211> 883
<212> PRT
<213> Homo sapiens

<400> 9

Ala His Pro Gly Arg Pro Arg Ala Val Pro Thr Gln Cys Asp Val Pro
1 5 10 15

Pro Asn Ser Arg Phe Asp Cys Ala Pro Asp Lys Ala Ile Thr Gln Glu
20 25 30

Gln Cys Glu Ala Arg Gly Cys Cys Tyr Ile Pro Ala Lys Gln Gly Leu
35 40 45

Gln Gly Ala Gln Met Gly Gln Pro Trp Cys Phe Phe Pro Pro Ser Tyr
50 55 60

Pro Ser Tyr Lys Leu Glu Asn Leu Ser Ser Ser Glu Met Gly Tyr Thr
65 70 75 80

Ala Thr Leu Thr Arg Thr Thr Pro Thr Phe Phe Pro Lys Asp Ile Leu
85 90 95

Thr Leu Arg Leu Asp Val Met Met Glu Thr Glu Asn Arg Leu His Phe
100 105 110

Thr Ile Lys Asp Pro Ala Asn Arg Arg Tyr Glu Val Pro Leu Glu Thr
115 120 125

Pro His Val His Ser Arg Ala Pro Ser Pro Leu Tyr Ser Val Glu Phe
130 135 140

Ser Glu Glu Pro Phe Gly Val Ile Val Arg Arg Gln Leu Asp Gly Arg
145 150 155 160

Val Leu Leu Asn Thr Thr Val Ala Pro Leu Phe Phe Ala Asp Gln Phe
165 170 175

Leu Gln Leu Ser Thr Ser Leu Pro Ser Gln Tyr Ile Thr Gly Leu Ala
180 185 190

Glu His Leu Ser Pro Leu Met Leu Ser Thr Ser Trp Thr Arg Ile Thr
195 200 205

Leu Trp Asn Arg Asp Leu Ala Pro Thr Pro Gly Ala Asn Leu Tyr Gly
210 215 220

Ser His Pro Phe Tyr Leu Ala Leu Glu Asp Gly Gly Ser Ala His Gly
225 230 235 240

Val Phe Leu Leu Asn Ser Asn Ala Met Asp Val Val Leu Gln Pro Ser
245 250 255

Pro Ala Leu Ser Trp Arg Ser Thr Gly Gly Ile Leu Asp Val Tyr Ile
260 265 270

Phe Leu Gly Pro Glu Pro Lys Ser Val Val Gln Gln Tyr Leu Asp Val
275 280 285

Val Gly Tyr Pro Phe Met Pro Pro Tyr Trp Gly Leu Gly Phe His Leu
290 295 300

```
Cys Arg Trp Gly Tyr Ser Ser Thr Ala Ile Thr Arg Gln Val Val Glu
305                 310                 315                 320

Asn Met Thr Arg Ala His Phe Pro Leu Asp Val Gln Trp Asn Asp Leu
                325                 330                 335

Asp Tyr Met Asp Ser Arg Arg Asp Phe Thr Phe Asn Lys Asp Gly Phe
            340                 345                 350

Arg Asp Phe Pro Ala Met Val Gln Glu Leu His Gln Gly Gly Arg Arg
        355                 360                 365

Tyr Met Met Ile Val Asp Pro Ala Ile Ser Ser Ser Gly Pro Ala Gly
    370                 375                 380

Ser Tyr Arg Pro Tyr Asp Glu Gly Leu Arg Arg Gly Val Phe Ile Thr
385                 390                 395                 400

Asn Glu Thr Gly Gln Pro Leu Ile Gly Lys Val Trp Pro Gly Ser Thr
                405                 410                 415

Ala Phe Pro Asp Phe Thr Asn Pro Thr Ala Leu Ala Trp Trp Glu Asp
            420                 425                 430

Met Val Ala Glu Phe His Asp Gln Val Pro Phe Asp Gly Met Trp Ile
        435                 440                 445

Asp Met Asn Glu Pro Ser Asn Phe Ile Arg Gly Ser Glu Asp Gly Cys
    450                 455                 460

Pro Asn Asn Glu Leu Glu Asn Pro Pro Tyr Val Pro Gly Val Val Gly
465                 470                 475                 480

Gly Thr Leu Gln Ala Ala Thr Ile Cys Ala Ser Ser His Gln Phe Leu
                485                 490                 495

Ser Thr His Tyr Asn Leu His Asn Leu Tyr Gly Leu Thr Glu Ala Ile
            500                 505                 510

Ala Ser His Arg Ala Leu Val Lys Ala Arg Gly Thr Arg Pro Phe Val
        515                 520                 525

Ile Ser Arg Ser Thr Phe Ala Gly His Gly Arg Tyr Ala Gly His Trp
    530                 535                 540

Thr Gly Asp Val Trp Ser Ser Trp Glu Gln Leu Ala Ser Ser Val Pro
545                 550                 555                 560

Glu Ile Leu Gln Phe Asn Leu Leu Gly Val Pro Leu Val Gly Ala Asp
                565                 570                 575
```

```
Val Cys Gly Phe Leu Gly Asn Thr Ser Glu Glu Leu Cys Val Arg Trp
            580                 585                 590

Thr Gln Leu Gly Ala Phe Tyr Pro Phe Met Arg Asn His Asn Ser Leu
        595                 600                 605

Leu Ser Leu Pro Gln Glu Pro Tyr Ser Phe Ser Glu Pro Ala Gln Gln
    610                 615                 620

Ala Met Arg Lys Ala Leu Thr Leu Arg Tyr Ala Leu Leu Pro His Leu
625                 630                 635                     640

Tyr Thr Leu Phe His Gln Ala His Val Ala Gly Glu Thr Val Ala Arg
                645                 650                 655

Pro Leu Phe Leu Glu Phe Pro Lys Asp Ser Ser Thr Trp Thr Val Asp
            660                 665                 670

His Gln Leu Leu Trp Gly Glu Ala Leu Leu Ile Thr Pro Val Leu Gln
        675                 680                 685

Ala Gly Lys Ala Glu Val Thr Gly Tyr Phe Pro Leu Gly Thr Trp Tyr
    690                 695                 700

Asp Leu Gln Thr Val Pro Val Glu Ala Leu Gly Ser Leu Pro Pro Pro
705                 710                 715                     720

Pro Ala Ala Pro Arg Glu Pro Ala Ile His Ser Glu Gly Gln Trp Val
                725                 730                 735

Thr Leu Pro Ala Pro Leu Asp Thr Ile Asn Val His Leu Arg Ala Gly
            740                 745                 750

Tyr Ile Ile Pro Leu Gln Gly Pro Gly Leu Thr Thr Thr Glu Ser Arg
        755                 760                 765

Gln Gln Pro Met Ala Leu Ala Val Ala Leu Thr Lys Gly Gly Glu Ala
    770                 775                 780

Arg Gly Glu Leu Phe Trp Asp Asp Gly Glu Ser Leu Glu Val Leu Glu
785                 790                 795                     800

Arg Gly Ala Tyr Thr Gln Val Ile Phe Leu Ala Arg Asn Asn Thr Ile
                805                 810                 815

Val Asn Glu Leu Val Arg Val Thr Ser Glu Gly Ala Gly Leu Gln Leu
            820                 825                 830

Gln Lys Val Thr Val Leu Gly Val Ala Thr Ala Pro Gln Gln Val Leu
        835                 840                 845
```

Ser Asn Gly Val Pro Val Ser Asn Phe Thr Tyr Ser Pro Asp Thr Lys
850 855 860

Val Leu Asp Ile Cys Val Ser Leu Leu Met Gly Glu Gln Phe Leu Val
865 870 875 880

Ser Trp Cys

<210> 10
<211> 626
<212> PRT
<213> Homo sapiens

<400> 10

Ala Pro His Leu Val His Val Asp Ala Ala Arg Ala Leu Trp Pro Leu
1 5 10 15

Arg Arg Phe Trp Arg Ser Thr Gly Phe Cys Pro Pro Leu Pro His Ser
20 25 30

Gln Ala Asp Gln Tyr Val Leu Ser Trp Asp Gln Gln Leu Asn Leu Ala
35 40 45

Tyr Val Gly Ala Val Pro His Arg Gly Ile Lys Gln Val Arg Thr His
50 55 60

Trp Leu Leu Glu Leu Val Thr Thr Arg Gly Ser Thr Gly Arg Gly Leu
65 70 75 80

Ser Tyr Asn Phe Thr His Leu Asp Gly Tyr Leu Asp Leu Leu Arg Glu
85 90 95

Asn Gln Leu Leu Pro Gly Phe Glu Leu Met Gly Ser Ala Ser Gly His
100 105 110

Phe Thr Asp Phe Glu Asp Lys Gln Gln Val Phe Glu Trp Lys Asp Leu
115 120 125

Val Ser Ser Leu Ala Arg Arg Tyr Ile Gly Arg Tyr Gly Leu Ala His
130 135 140

Val Ser Lys Trp Asn Phe Glu Thr Trp Asn Glu Pro Asp His His Asp
145 150 155 160

Phe Asp Asn Val Ser Met Thr Met Gln Gly Phe Leu Asn Tyr Tyr Asp
165 170 175

Ala Cys Ser Glu Gly Leu Arg Ala Ala Ser Pro Ala Leu Arg Leu Gly
180 185 190

```
Gly Pro Gly Asp Ser Phe His Thr Pro Pro Arg Ser Pro Leu Ser Trp
        195                 200                 205

Gly Leu Leu Arg His Cys His Asp Gly Thr Asn Phe Phe Thr Gly Glu
    210                 215                 220

Ala Gly Val Arg Leu Asp Tyr Ile Ser Leu His Arg Lys Gly Ala Arg
225                 230                 235                 240

Ser Ser Ile Ser Ile Leu Glu Gln Glu Lys Val Val Ala Gln Gln Ile
                245                 250                 255

Arg Gln Leu Phe Pro Lys Phe Ala Asp Thr Pro Ile Tyr Asn Asp Glu
            260                 265                 270

Ala Asp Pro Leu Val Gly Trp Ser Leu Pro Gln Pro Trp Arg Ala Asp
        275                 280                 285

Val Thr Tyr Ala Ala Met Val Val Lys Val Ile Ala Gln His Gln Asn
    290                 295                 300

Leu Leu Leu Ala Asn Thr Thr Ser Ala Phe Pro Tyr Ala Leu Leu Ser
305                 310                 315                 320

Asn Asp Asn Ala Phe Leu Ser Tyr His Pro His Pro Phe Ala Gln Arg
                325                 330                 335

Thr Leu Thr Ala Arg Phe Gln Val Asn Asn Thr Arg Pro Pro His Val
            340                 345                 350

Gln Leu Leu Arg Lys Pro Val Leu Thr Ala Met Gly Leu Leu Ala Leu
        355                 360                 365

Leu Asp Glu Glu Gln Leu Trp Ala Glu Val Ser Gln Ala Gly Thr Val
    370                 375                 380

Leu Asp Ser Asn His Thr Val Gly Val Leu Ala Ser Ala His Arg Pro
385                 390                 395                 400

Gln Gly Pro Ala Asp Ala Trp Arg Ala Ala Val Leu Ile Tyr Ala Ser
                405                 410                 415

Asp Asp Thr Arg Ala His Pro Asn Arg Ser Val Ala Val Thr Leu Arg
            420                 425                 430

Leu Arg Gly Val Pro Pro Gly Pro Gly Leu Val Tyr Val Thr Arg Tyr
        435                 440                 445

Leu Asp Asn Gly Leu Cys Ser Pro Asp Gly Glu Trp Arg Arg Leu Gly
    450                 455                 460
```

Arg Pro Val Phe Pro Thr Ala Glu Gln Phe Arg Arg Met Arg Ala Ala
465 470 475 480

Glu Asp Pro Val Ala Ala Ala Pro Arg Pro Leu Pro Ala Gly Gly Arg
485 490 495

Leu Thr Leu Arg Pro Ala Leu Arg Leu Pro Ser Leu Leu Leu Val His
500 505 510

Val Cys Ala Arg Pro Glu Lys Pro Pro Gly Gln Val Thr Arg Leu Arg
515 520 525

Ala Leu Pro Leu Thr Gln Gly Gln Leu Val Leu Val Trp Ser Asp Glu
530 535 540

His Val Gly Ser Lys Cys Leu Trp Thr Tyr Glu Ile Gln Phe Ser Gln
545 550 555 560

Asp Gly Lys Ala Tyr Thr Pro Val Ser Arg Lys Pro Ser Thr Phe Asn
565 570 575

Leu Phe Val Phe Ser Pro Asp Thr Gly Ala Val Ser Gly Ser Tyr Arg
580 585 590

Val Arg Ala Leu Asp Tyr Trp Ala Arg Pro Gly Pro Phe Ser Asp Pro
595 600 605

Val Pro Tyr Leu Glu Val Pro Val Pro Arg Gly Pro Pro Ser Pro Gly
610 615 620

Asn Pro
625

<210> 11
<211> 517
<212> PRT
<213> Homo sapiens

<400> 11

Thr Asp Ala Leu Asn Val Leu Leu Ile Ile Val Asp Asp Leu Arg Pro
1 5 10 15

Ser Leu Gly Cys Tyr Gly Asp Lys Leu Val Arg Ser Pro Asn Ile Asp
20 25 30

Gln Leu Ala Ser His Ser Leu Leu Phe Gln Asn Ala Phe Ala Gln Gln
35 40 45

Ala Val Cys Ala Pro Ser Arg Val Ser Phe Leu Thr Gly Arg Arg Pro
50 55 60

Asp Thr Thr Arg Leu Tyr Asp Phe Asn Ser Tyr Trp Arg Val His Ala

65 70 75 80

Gly Asn Phe Ser Thr Ile Pro Gln Tyr Phe Lys Glu Asn Gly Tyr Val
85 90 95

Thr Met Ser Val Gly Lys Val Phe His Pro Gly Ile Ser Ser Asn His
100 105 110

Thr Asp Asp Ser Pro Tyr Ser Trp Ser Phe Pro Pro Tyr His Pro Ser
115 120 125

Ser Glu Lys Tyr Glu Asn Thr Lys Thr Cys Arg Gly Pro Asp Gly Glu
130 135 140

Leu His Ala Asn Leu Leu Cys Pro Val Asp Val Leu Asp Val Pro Glu
145 150 155 160

Gly Thr Leu Pro Asp Lys Gln Ser Thr Glu Gln Ala Ile Gln Leu Leu
165 170 175

Glu Lys Met Lys Thr Ser Ala Ser Pro Phe Phe Leu Ala Val Gly Tyr
180 185 190

His Lys Pro His Ile Pro Phe Arg Tyr Pro Lys Glu Phe Gln Lys Leu
195 200 205

Tyr Pro Leu Glu Asn Ile Thr Leu Ala Pro Asp Pro Glu Val Pro Asp
210 215 220

Gly Leu Pro Pro Val Ala Tyr Asn Pro Trp Met Asp Ile Arg Gln Arg
225 230 235 240

Glu Asp Val Gln Ala Leu Asn Ile Ser Val Pro Tyr Gly Pro Ile Pro
245 250 255

Val Asp Phe Gln Arg Lys Ile Arg Gln Ser Tyr Phe Ala Ser Val Ser
260 265 270

Tyr Leu Asp Thr Gln Val Gly Arg Leu Leu Ser Ala Leu Asp Asp Leu
275 280 285

Gln Leu Ala Asn Ser Thr Ile Ile Ala Phe Thr Ser Asp His Gly Trp
290 295 300

Ala Leu Gly Glu His Gly Glu Trp Ala Lys Tyr Ser Asn Phe Asp Val
305 310 315 320

Ala Thr His Val Pro Leu Ile Phe Tyr Val Pro Gly Arg Thr Ala Ser
325 330 335

Leu Pro Glu Ala Gly Glu Lys Leu Phe Pro Tyr Leu Asp Pro Phe Asp

```
            340                 345                 350

Ser Ala Ser Gln Leu Met Glu Pro Gly Arg Gln Ser Met Asp Leu Val
        355                 360                 365

Glu Leu Val Ser Leu Phe Pro Thr Leu Ala Gly Leu Ala Gly Leu Gln
    370                 375                 380

Val Pro Pro Arg Cys Pro Val Pro Ser Phe His Val Glu Leu Cys Arg
385                 390                 395                 400

Glu Gly Lys Asn Leu Leu Lys His Phe Arg Phe Arg Asp Leu Glu Glu
                405                 410                 415

Asp Pro Tyr Leu Pro Gly Asn Pro Arg Glu Leu Ile Ala Tyr Ser Gln
            420                 425                 430

Tyr Pro Arg Pro Ser Asp Ile Pro Gln Trp Asn Ser Asp Lys Pro Ser
        435                 440                 445

Leu Lys Asp Ile Lys Ile Met Gly Tyr Ser Ile Arg Thr Ile Asp Tyr
    450                 455                 460

Arg Tyr Thr Val Trp Val Gly Phe Asn Pro Asp Glu Phe Leu Ala Asn
465                 470                 475                 480

Phe Ser Asp Ile His Ala Gly Glu Leu Tyr Phe Val Asp Ser Asp Pro
                485                 490                 495

Leu Gln Asp His Asn Met Tyr Asn Asp Ser Gln Gly Gly Asp Leu Phe
            500                 505                 510

Gln Leu Leu Met Pro
        515
```

<210> 12
<211> 497
<212> PRT
<213> Homo sapiens

<400> 12

```
Ser Gly Ala Gly Ala Ser Arg Pro Pro His Leu Val Phe Leu Leu Ala
1               5                   10                  15

Asp Asp Leu Gly Trp Asn Asp Val Gly Phe His Gly Ser Arg Ile Arg
            20                  25                  30

Thr Pro His Leu Asp Ala Leu Ala Ala Gly Gly Val Leu Leu Asp Asn
        35                  40                  45

Tyr Tyr Thr Gln Pro Leu Cys Thr Pro Ser Arg Ser Gln Leu Leu Thr
    50                  55                  60
```

```
Gly Arg Tyr Gln Ile Arg Thr Gly Leu Gln His Gln Ile Ile Trp Pro
65                  70                  75                  80

Cys Gln Pro Ser Cys Val Pro Leu Asp Glu Lys Leu Leu Pro Gln Leu
                85                  90                  95

Leu Lys Glu Ala Gly Tyr Thr Thr His Met Val Gly Lys Trp His Leu
            100                 105                 110

Gly Met Tyr Arg Lys Glu Cys Leu Pro Thr Arg Arg Gly Phe Asp Thr
        115                 120                 125

Tyr Phe Gly Tyr Leu Leu Gly Ser Glu Asp Tyr Tyr Ser His Glu Arg
    130                 135                 140

Cys Thr Leu Ile Asp Ala Leu Asn Val Thr Arg Cys Ala Leu Asp Phe
145                 150                 155                 160

Arg Asp Gly Glu Glu Val Ala Thr Gly Tyr Lys Asn Met Tyr Ser Thr
                165                 170                 175

Asn Ile Phe Thr Lys Arg Ala Ile Ala Leu Ile Thr Asn His Pro Pro
            180                 185                 190

Glu Lys Pro Leu Phe Leu Tyr Leu Ala Leu Gln Ser Val His Glu Pro
        195                 200                 205

Leu Gln Val Pro Glu Glu Tyr Leu Lys Pro Tyr Asp Phe Ile Gln Asp
    210                 215                 220

Lys Asn Arg His His Tyr Ala Gly Met Val Ser Leu Met Asp Glu Ala
225                 230                 235                 240

Val Gly Asn Val Thr Ala Ala Leu Lys Ser Ser Gly Leu Trp Asn Asn
                245                 250                 255

Thr Val Phe Ile Phe Ser Thr Asp Asn Gly Gly Gln Thr Leu Ala Gly
            260                 265                 270

Gly Asn Asn Trp Pro Leu Arg Gly Arg Lys Trp Ser Leu Trp Glu Gly
        275                 280                 285

Gly Val Arg Gly Val Gly Phe Val Ala Ser Pro Leu Leu Lys Gln Lys
    290                 295                 300

Gly Val Lys Asn Arg Glu Leu Ile His Ile Ser Asp Trp Leu Pro Thr
305                 310                 315                 320

Leu Val Lys Leu Ala Arg Gly His Thr Asn Gly Thr Lys Pro Leu Asp
                325                 330                 335
```

Gly Phe Asp Val Trp Lys Thr Ile Ser Glu Gly Ser Pro Ser Pro Arg
340 345 350

Ile Glu Leu Leu His Asn Ile Asp Pro Asn Phe Val Asp Ser Ser Pro
355 360 365

Cys Pro Arg Asn Ser Met Ala Pro Ala Lys Asp Asp Ser Ser Leu Pro
370 375 380

Glu Tyr Ser Ala Phe Asn Thr Ser Val His Ala Ala Ile Arg His Gly
385 390 395 400

Asn Trp Lys Leu Leu Thr Gly Tyr Pro Gly Cys Gly Tyr Trp Phe Pro
405 410 415

Pro Pro Ser Gln Tyr Asn Val Ser Glu Ile Pro Ser Ser Asp Pro Pro
420 425 430

Thr Lys Thr Leu Trp Leu Phe Asp Ile Asp Arg Asp Pro Glu Glu Arg
435 440 445

His Asp Leu Ser Arg Glu Tyr Pro His Ile Val Thr Lys Leu Leu Ser
450 455 460

Arg Leu Gln Phe Tyr His Lys His Ser Val Pro Val Tyr Phe Pro Ala
465 470 475 480

Gln Asp Pro Arg Cys Asp Pro Lys Ala Thr Gly Val Trp Gly Pro Trp
485 490 495

Met

<210> 13
<211> 583
<212> PRT
<213> Homo sapiens

<400> 13

Leu Ser Asp Ser Arg Val Leu Trp Ala Pro Ala Glu Ala His Pro Leu
1 5 10 15

Ser Pro Gln Gly His Pro Ala Arg Leu His Arg Ile Val Pro Arg Leu
20 25 30

Arg Asp Val Phe Gly Trp Gly Asn Leu Thr Cys Pro Ile Cys Lys Gly
35 40 45

Leu Phe Thr Ala Ile Asn Leu Gly Leu Lys Lys Glu Pro Asn Val Ala
50 55 60

```
Arg Val Gly Ser Val Ala Ile Lys Leu Cys Asn Leu Leu Lys Ile Ala
65                  70                  75                  80

Pro Pro Ala Val Cys Gln Ser Ile Val His Leu Phe Glu Asp Asp Met
                85                  90                  95

Val Glu Val Trp Arg Arg Ser Val Leu Ser Pro Ser Glu Ala Cys Gly
            100                 105                 110

Leu Leu Leu Gly Ser Thr Cys Gly His Trp Asp Ile Phe Ser Ser Trp
        115                 120                 125

Asn Ile Ser Leu Pro Thr Val Pro Lys Pro Pro Pro Lys Pro Pro Ser
    130                 135                 140

Pro Pro Ala Pro Gly Ala Pro Val Ser Arg Ile Leu Phe Leu Thr Asp
145                 150                 155                 160

Leu His Trp Asp His Asp Tyr Leu Glu Gly Thr Asp Pro Asp Cys Ala
                165                 170                 175

Asp Pro Leu Cys Cys Arg Arg Gly Ser Gly Leu Pro Pro Ala Ser Arg
            180                 185                 190

Pro Gly Ala Gly Tyr Trp Gly Glu Tyr Ser Lys Cys Asp Leu Pro Leu
        195                 200                 205

Arg Thr Leu Glu Ser Leu Leu Ser Gly Leu Gly Pro Ala Gly Pro Phe
    210                 215                 220

Asp Met Val Tyr Trp Thr Gly Asp Ile Pro Ala His Asp Val Trp His
225                 230                 235                 240

Gln Thr Arg Gln Asp Gln Leu Arg Ala Leu Thr Thr Val Thr Ala Leu
                245                 250                 255

Val Arg Lys Phe Leu Gly Pro Val Pro Val Tyr Pro Ala Val Gly Asn
            260                 265                 270

His Glu Ser Thr Pro Val Asn Ser Phe Pro Pro Pro Phe Ile Glu Gly
        275                 280                 285

Asn His Ser Ser Arg Trp Leu Tyr Glu Ala Met Ala Lys Ala Trp Glu
    290                 295                 300

Pro Trp Leu Pro Ala Glu Ala Leu Arg Thr Leu Arg Ile Gly Gly Phe
305                 310                 315                 320

Tyr Ala Leu Ser Pro Tyr Pro Gly Leu Arg Leu Ile Ser Leu Asn Met
                325                 330                 335
```

Asn Phe Cys Ser Arg Glu Asn Phe Trp Leu Leu Ile Asn Ser Thr Asp
340 345 350

Pro Ala Gly Gln Leu Gln Trp Leu Val Gly Glu Leu Gln Ala Ala Glu
355 360 365

Asp Arg Gly Asp Lys Val His Ile Ile Gly His Ile Pro Pro Gly His
370 375 380

Cys Leu Lys Ser Trp Ser Trp Asn Tyr Tyr Arg Ile Val Ala Arg Tyr
385 390 395 400

Glu Asn Thr Leu Ala Ala Gln Phe Phe Gly His Thr His Val Asp Glu
405 410 415

Phe Glu Val Phe Tyr Asp Glu Glu Thr Leu Ser Arg Pro Leu Ala Val
420 425 430

Ala Phe Leu Ala Pro Ser Ala Thr Thr Tyr Ile Gly Leu Asn Pro Gly
435 440 445

Tyr Arg Val Tyr Gln Ile Asp Gly Asn Tyr Ser Gly Ser Ser His Val
450 455 460

Val Leu Asp His Glu Thr Tyr Ile Leu Asn Leu Thr Gln Ala Asn Ile
465 470 475 480

Pro Gly Ala Ile Pro His Trp Gln Leu Leu Tyr Arg Ala Arg Glu Thr
485 490 495

Tyr Gly Leu Pro Asn Thr Leu Pro Thr Ala Trp His Asn Leu Val Tyr
500 505 510

Arg Met Arg Gly Asp Met Gln Leu Phe Gln Thr Phe Trp Phe Leu Tyr
515 520 525

His Lys Gly His Pro Pro Ser Glu Pro Cys Gly Thr Pro Cys Arg Leu
530 535 540

Ala Thr Leu Cys Ala Gln Leu Ser Ala Arg Ala Asp Ser Pro Ala Leu
545 550 555 560

Cys Arg His Leu Met Pro Asp Gly Ser Leu Pro Glu Ala Gln Ser Leu
565 570 575

Trp Pro Arg Pro Leu Phe Cys
580

<210> 14
<211> 378
<212> PRT

<213> Homo sapiens

<400> 14

Ser Gly Gly Lys Leu Thr Ala Val Asp Pro Glu Thr Asn Met Asn Val
1 5 10 15

Ser Glu Ile Ile Ser Tyr Trp Gly Phe Pro Ser Glu Glu Tyr Leu Val
20 25 30

Glu Thr Glu Asp Gly Tyr Ile Leu Cys Leu Asn Arg Ile Pro His Gly
35 40 45

Arg Lys Asn His Ser Asp Lys Gly Pro Lys Pro Val Val Phe Leu Gln
50 55 60

His Gly Leu Leu Ala Asp Ser Ser Asn Trp Val Thr Asn Leu Ala Asn
65 70 75 80

Ser Ser Leu Gly Phe Ile Leu Ala Asp Ala Gly Phe Asp Val Trp Met
85 90 95

Gly Asn Ser Arg Gly Asn Thr Trp Ser Arg Lys His Lys Thr Leu Ser
100 105 110

Val Ser Gln Asp Glu Phe Trp Ala Phe Ser Tyr Asp Glu Met Ala Lys
115 120 125

Tyr Asp Leu Pro Ala Ser Ile Asn Phe Ile Leu Asn Lys Thr Gly Gln
130 135 140

Glu Gln Val Tyr Tyr Val Gly His Ser Gln Gly Thr Thr Ile Gly Phe
145 150 155 160

Ile Ala Phe Ser Gln Ile Pro Glu Leu Ala Lys Arg Ile Lys Met Phe
165 170 175

Phe Ala Leu Gly Pro Val Ala Ser Val Ala Phe Cys Thr Ser Pro Met
180 185 190

Ala Lys Leu Gly Arg Leu Pro Asp His Leu Ile Lys Asp Leu Phe Gly
195 200 205

Asp Lys Glu Phe Leu Pro Gln Ser Ala Phe Leu Lys Trp Leu Gly Thr
210 215 220

His Val Cys Thr His Val Ile Leu Lys Glu Leu Cys Gly Asn Leu Cys
225 230 235 240

Phe Leu Leu Cys Gly Phe Asn Glu Arg Asn Leu Asn Met Ser Arg Val
245 250 255

Asp Val Tyr Thr Thr His Ser Pro Ala Gly Thr Ser Val Gln Asn Met
260 265 270

Leu His Trp Ser Gln Ala Val Lys Phe Gln Lys Phe Gln Ala Phe Asp
275 280 285

Trp Gly Ser Ser Ala Lys Asn Tyr Phe His Tyr Asn Gln Ser Tyr Pro
290 295 300

Pro Thr Tyr Asn Val Lys Asp Met Leu Val Pro Thr Ala Val Trp Ser
305 310 315 320

Gly Gly His Asp Trp Leu Ala Asp Val Tyr Asp Val Asn Ile Leu Leu
325 330 335

Thr Gln Ile Thr Asn Leu Val Phe His Glu Ser Ile Pro Glu Trp Glu
340 345 350

His Leu Asp Phe Ile Trp Gly Leu Asp Ala Pro Trp Arg Leu Tyr Asn
355 360 365

Lys Ile Ile Asn Leu Met Arg Lys Tyr Gln
370 375

<210> 15
<211> 483
<212> PRT
<213> Human immunodeficiency virus 1

<400> 15

Ser Ala Thr Glu Lys Leu Trp Val Thr Val Tyr Tyr Gly Val Pro Val
1 5 10 15

Trp Lys Glu Ala Thr Thr Thr Leu Phe Cys Ala Ser Asp Ala Lys Ala
20 25 30

Tyr Asp Thr Glu Val His Asn Val Trp Ala Thr His Ala Cys Val Pro
35 40 45

Thr Asp Pro Asn Pro Gln Glu Val Val Leu Val Asn Val Thr Glu Asn
50 55 60

Phe Asn Met Trp Lys Asn Asp Met Val Glu Gln Met His Glu Asp Ile
65 70 75 80

Ile Ser Leu Trp Asp Gln Ser Leu Lys Pro Cys Val Lys Leu Thr Pro
85 90 95

Leu Cys Val Ser Leu Lys Cys Thr Asp Leu Lys Asn Asp Thr Asn Thr
100 105 110

Asn Ser Ser Ser Gly Arg Met Ile Met Glu Lys Gly Glu Ile Lys Asn

115 120 125

Cys Ser Phe Asn Ile Ser Thr Ser Ile Arg Gly Lys Val Gln Lys Glu
130 135 140

Tyr Ala Phe Phe Tyr Lys Leu Asp Ile Ile Pro Ile Asp Asn Asp Thr
145 150 155 160

Thr Ser Tyr Lys Leu Thr Ser Cys Asn Thr Ser Val Ile Thr Gln Ala
165 170 175

Cys Pro Lys Val Ser Phe Glu Pro Ile Pro Ile His Tyr Cys Ala Pro
180 185 190

Ala Gly Phe Ala Ile Leu Lys Cys Asn Asn Lys Thr Phe Asn Gly Thr
195 200 205

Gly Pro Cys Thr Asn Val Ser Thr Val Gln Cys Thr His Gly Ile Arg
210 215 220

Pro Val Val Ser Thr Gln Leu Leu Leu Asn Gly Ser Leu Ala Glu Glu
225 230 235 240

Glu Val Val Ile Arg Ser Val Asn Phe Thr Asp Asn Ala Lys Thr Ile
245 250 255

Ile Val Gln Leu Asn Thr Ser Val Glu Ile Asn Cys Thr Arg Pro Asn
260 265 270

Asn Asn Thr Arg Lys Arg Ile Arg Ile Gln Arg Gly Pro Gly Arg Ala
275 280 285

Phe Val Thr Ile Gly Lys Ile Gly Asn Met Arg Gln Ala His Cys Asn
290 295 300

Ile Ser Arg Ala Lys Trp Asn Asn Thr Leu Lys Gln Ile Ala Ser Lys
305 310 315 320

Leu Arg Glu Gln Phe Gly Asn Asn Lys Thr Ile Ile Phe Lys Gln Ser
325 330 335

Ser Gly Gly Asp Pro Glu Ile Val Thr His Ser Phe Asn Cys Gly Gly
340 345 350

Glu Phe Phe Tyr Cys Asn Ser Thr Gln Leu Phe Asn Ser Thr Trp Phe
355 360 365

Asn Ser Thr Trp Ser Thr Glu Gly Ser Asn Asn Thr Glu Gly Ser Asp
370 375 380

Thr Ile Thr Leu Pro Cys Arg Ile Lys Gln Ile Ile Asn Met Trp Gln

385 390 395 400

Lys Val Gly Lys Ala Met Tyr Ala Pro Pro Ile Ser Gly Gln Ile Arg
405 410 415

Cys Ser Ser Asn Ile Thr Gly Leu Leu Leu Thr Arg Asp Gly Gly Asn
420 425 430

Ser Asn Asn Glu Ser Glu Ile Phe Arg Pro Gly Gly Gly Asp Met Arg
435 440 445

Asp Asn Trp Arg Ser Glu Leu Tyr Lys Tyr Lys Val Val Lys Ile Glu
450 455 460

Pro Leu Gly Val Ala Pro Thr Lys Ala Lys Arg Arg Val Val Gln Arg
465 470 475 480

Glu Lys Arg

<210> 16
<211> 345
<212> PRT
<213> Human immunodeficiency virus 1

<400> 16

Ala Val Gly Ile Gly Ala Leu Phe Leu Gly Phe Leu Gly Ala Ala Gly
1 5 10 15

Ser Thr Met Gly Ala Ala Ser Met Thr Leu Thr Val Gln Ala Arg Gln
20 25 30

Leu Leu Ser Gly Ile Val Gln Gln Gln Asn Asn Leu Leu Arg Ala Ile
35 40 45

Glu Ala Gln Gln His Leu Leu Gln Leu Thr Val Trp Gly Ile Lys Gln
50 55 60

Leu Gln Ala Arg Ile Leu Ala Val Glu Arg Tyr Leu Lys Asp Gln Gln
65 70 75 80

Leu Leu Gly Ile Trp Gly Cys Ser Gly Lys Leu Ile Cys Thr Thr Ala
85 90 95

Val Pro Trp Asn Ala Ser Trp Ser Asn Lys Ser Leu Glu Gln Ile Trp
100 105 110

Asn His Thr Thr Trp Met Glu Trp Asp Arg Glu Ile Asn Asn Tyr Thr
115 120 125

Ser Leu Ile His Ser Leu Ile Glu Glu Ser Gln Asn Gln Gln Glu Lys
130 135 140

Asn Glu Gln Glu Leu Leu Glu Leu Asp Lys Trp Ala Ser Leu Trp Asn
145 150 155 160

Trp Phe Asn Ile Thr Asn Trp Leu Trp Tyr Ile Lys Leu Phe Ile Met
165 170 175

Ile Val Gly Gly Leu Val Gly Leu Arg Ile Val Phe Ala Val Leu Ser
180 185 190

Ile Val Asn Arg Val Arg Gln Gly Tyr Ser Pro Leu Ser Phe Gln Thr
195 200 205

His Leu Pro Thr Pro Arg Gly Pro Asp Arg Pro Glu Gly Ile Glu Glu
210 215 220

Glu Gly Gly Glu Arg Asp Arg Asp Arg Ser Ile Arg Leu Val Asn Gly
225 230 235 240

Ser Leu Ala Leu Ile Trp Asp Asp Leu Arg Ser Leu Cys Leu Phe Ser
245 250 255

Tyr His Arg Leu Arg Asp Leu Leu Leu Ile Val Thr Arg Ile Val Glu
260 265 270

Leu Leu Gly Arg Arg Gly Trp Glu Ala Leu Lys Tyr Trp Trp Asn Leu
275 280 285

Leu Gln Tyr Trp Ser Gln Glu Leu Lys Asn Ser Ala Val Ser Leu Leu
290 295 300

Asn Ala Thr Ala Ile Ala Val Ala Glu Gly Thr Asp Arg Val Ile Glu
305 310 315 320

Val Val Gln Gly Ala Cys Arg Ala Ile Arg His Ile Pro Arg Arg Ile
325 330 335

Arg Gln Gly Leu Glu Arg Ile Leu Leu
340 345

<210> 17
<211> 192
<212> PRT
<213> Hepatitis C virus

<400> 17

Tyr Gln Val Arg Asn Ser Ser Gly Leu Tyr His Val Thr Asn Asp Cys
1 5 10 15

Pro Asn Ser Ser Val Val Tyr Glu Ala Ala Asp Ala Ile Leu His Thr
20 25 30

Pro Gly Cys Val Pro Cys Val Arg Glu Gly Asn Ala Ser Arg Cys Trp
35 40 45

Val Ala Val Thr Pro Thr Val Ala Thr Arg Asp Gly Lys Leu Pro Thr
50 55 60

Thr Gln Leu Arg Arg His Ile Asp Leu Leu Val Gly Ser Ala Thr Leu
65 70 75 80

Cys Ser Ala Leu Tyr Val Gly Asp Leu Cys Gly Ser Val Phe Leu Val
85 90 95

Gly Gln Leu Phe Thr Phe Ser Pro Arg His His Trp Thr Thr Gln Asp
100 105 110

Cys Asn Cys Ser Ile Tyr Pro Gly His Ile Thr Gly His Arg Met Ala
115 120 125

Trp Asn Met Met Met Asn Trp Ser Pro Thr Ala Ala Leu Val Val Ala
130 135 140

Gln Leu Leu Arg Ile Pro Gln Ala Ile Met Asp Met Ile Ala Gly Ala
145 150 155 160

His Trp Gly Val Leu Ala Gly Ile Lys Tyr Phe Ser Met Val Gly Asn
165 170 175

Trp Ala Lys Val Leu Val Val Leu Leu Leu Phe Ala Gly Val Asp Ala
180 185 190

<210> 18
<211> 363
<212> PRT
<213> Hepatitis C virus

<400> 18

Glu Thr His Val Thr Gly Gly Asn Ala Gly Arg Thr Thr Ala Gly Leu
1 5 10 15

Val Gly Leu Leu Thr Pro Gly Ala Lys Gln Asn Ile Gln Leu Ile Asn
20 25 30

Thr Asn Gly Ser Trp His Ile Asn Ser Thr Ala Leu Asn Cys Asn Glu
35 40 45

Ser Leu Asn Thr Gly Trp Leu Ala Gly Leu Phe Tyr Gln His Lys Phe
50 55 60

Asn Ser Ser Gly Cys Pro Glu Arg Leu Ala Ser Cys Arg Arg Leu Thr
65 70 75 80

Asp Phe Ala Gln Gly Trp Gly Pro Ile Ser Tyr Ala Asn Gly Ser Gly
85 90 95

Leu Asp Glu Arg Pro Tyr Cys Trp His Tyr Pro Pro Arg Pro Cys Gly
100 105 110

Ile Val Pro Ala Lys Ser Val Cys Gly Pro Val Tyr Cys Phe Thr Pro
115 120 125

Ser Pro Val Val Val Gly Thr Thr Asp Arg Ser Gly Ala Pro Thr Tyr
130 135 140

Ser Trp Gly Ala Asn Asp Thr Asp Val Phe Val Leu Asn Asn Thr Arg
145 150 155 160

Pro Pro Leu Gly Asn Trp Phe Gly Cys Thr Trp Met Asn Ser Thr Gly
165 170 175

Phe Thr Lys Val Cys Gly Ala Pro Pro Cys Val Ile Gly Gly Val Gly
180 185 190

Asn Asn Thr Leu Leu Cys Pro Thr Asp Cys Phe Arg Lys Tyr Pro Glu
195 200 205

Ala Thr Tyr Ser Arg Cys Gly Ser Gly Pro Arg Ile Thr Pro Arg Cys
210 215 220

Met Val Asp Tyr Pro Tyr Arg Leu Trp His Tyr Pro Cys Thr Ile Asn
225 230 235 240

Tyr Thr Ile Phe Lys Val Arg Met Tyr Val Gly Gly Val Glu His Arg
245 250 255

Leu Glu Ala Ala Cys Asn Trp Thr Arg Gly Glu Arg Cys Asp Leu Glu
260 265 270

Asp Arg Asp Arg Ser Glu Leu Ser Pro Leu Leu Leu Ser Thr Thr Gln
275 280 285

Trp Gln Val Leu Pro Cys Ser Phe Thr Thr Leu Pro Ala Leu Ser Thr
290 295 300

Gly Leu Ile His Leu His Gln Asn Ile Val Asp Val Gln Tyr Leu Tyr
305 310 315 320

Gly Val Gly Ser Ser Ile Ala Ser Trp Ala Ile Lys Trp Glu Tyr Val
325 330 335

Val Leu Leu Phe Leu Leu Leu Ala Asp Ala Arg Val Cys Ser Cys Leu
340 345 350

Trp Met Met Leu Leu Ile Ser Gln Ala Glu Ala
355 360

<210> 19
<211> 495
<212> PRT
<213> Dengue virus 1

<400> 19

Met Arg Cys Val Gly Ile Gly Asn Arg Asp Phe Val Glu Gly Leu Ser
1 5 10 15

Gly Ala Thr Trp Val Asp Val Val Leu Glu His Gly Ser Cys Val Thr
20 25 30

Thr Met Ala Lys Asn Lys Pro Thr Leu Asp Ile Glu Leu Leu Lys Thr
35 40 45

Glu Val Thr Asn Pro Ala Ile Leu Arg Lys Leu Cys Ile Glu Ala Lys
50 55 60

Ile Ser Asn Thr Thr Thr Asp Ser Arg Cys Pro Thr Gln Gly Glu Ala
65 70 75 80

Thr Leu Val Glu Glu Gln Asp Ala Asn Phe Val Cys Arg Arg Thr Phe
85 90 95

Val Asp Arg Gly Trp Gly Asn Gly Cys Gly Leu Phe Gly Lys Gly Ser
100 105 110

Leu Leu Thr Cys Ala Lys Phe Lys Cys Val Thr Lys Leu Glu Gly Lys
115 120 125

Ile Val Gln Tyr Glu Asn Leu Lys Tyr Ser Val Ile Val Thr Val His
130 135 140

Thr Gly Asp Gln His Gln Val Gly Asn Glu Thr Thr Glu His Gly Thr
145 150 155 160

Ile Ala Thr Ile Thr Pro Gln Ala Pro Thr Ser Glu Ile Gln Leu Thr
165 170 175

Asp Tyr Gly Ala Leu Thr Leu Asp Cys Ser Pro Arg Thr Gly Leu Asp
180 185 190

Phe Asn Glu Met Val Leu Leu Thr Met Lys Glu Lys Ser Trp Leu Val
195 200 205

His Lys Gln Trp Phe Leu Asp Leu Pro Leu Pro Trp Thr Ser Gly Ala
210 215 220

Ser Thr Ser Gln Glu Thr Trp Asn Arg Gln Asp Leu Leu Val Thr Phe

225 230 235 240

Lys Thr Ala His Ala Lys Lys Gln Glu Val Val Val Leu Gly Ser Gln
245 250 255

Glu Gly Ala Met His Thr Ala Leu Thr Gly Ala Thr Glu Ile Gln Thr
260 265 270

Ser Gly Thr Thr Thr Ile Phe Ala Gly His Leu Lys Cys Arg Leu Lys
275 280 285

Met Asp Lys Leu Thr Leu Lys Gly Thr Ser Tyr Val Met Cys Thr Gly
290 295 300

Ser Phe Lys Leu Glu Lys Glu Val Ala Glu Thr Gln His Gly Thr Val
305 310 315 320

Leu Val Gln Val Lys Tyr Glu Gly Thr Asp Ala Pro Cys Lys Ile Pro
325 330 335

Phe Ser Thr Gln Asp Glu Lys Gly Val Thr Gln Asn Gly Arg Leu Ile
340 345 350

Thr Ala Asn Pro Ile Val Thr Asp Lys Glu Lys Pro Val Asn Ile Glu
355 360 365

Thr Glu Pro Pro Phe Gly Glu Ser Tyr Ile Val Val Gly Ala Gly Glu
370 375 380

Lys Ala Leu Lys Leu Ser Trp Phe Lys Lys Gly Ser Ser Ile Gly Lys
385 390 395 400

Met Phe Glu Ala Thr Ala Arg Gly Ala Arg Arg Met Ala Ile Leu Gly
405 410 415

Asp Thr Ala Trp Asp Phe Gly Ser Ile Gly Gly Val Phe Thr Ser Val
420 425 430

Gly Lys Leu Val His Gln Val Phe Gly Thr Ala Tyr Gly Val Leu Phe
435 440 445

Ser Gly Val Ser Trp Thr Met Lys Ile Gly Ile Gly Ile Leu Leu Thr
450 455 460

Trp Leu Gly Leu Asn Ser Arg Ser Thr Ser Leu Ser Met Thr Cys Ile
465 470 475 480

Ala Val Gly Met Val Thr Leu Tyr Leu Gly Val Met Val Gln Ala
485 490 495

<210> 20

<211> 501
<212> PRT
<213> West Nile Virus

<400> 20

```
Phe Asn Cys Leu Gly Met Ser Asn Arg Asp Phe Leu Glu Gly Val Ser
1               5                   10                  15

Gly Ala Thr Trp Val Asp Leu Val Leu Glu Gly Asp Ser Cys Val Thr
            20                  25                  30

Ile Met Ser Lys Asp Lys Pro Thr Ile Asp Val Lys Met Met Asn Met
        35                  40                  45

Glu Ala Ala Asn Leu Ala Glu Val Arg Ser Tyr Cys Tyr Leu Ala Thr
    50                  55                  60

Val Ser Asp Leu Ser Thr Lys Ala Ala Cys Pro Thr Met Gly Glu Ala
65                  70                  75                  80

His Asn Asp Lys Arg Ala Asp Pro Ala Phe Val Cys Arg Gln Gly Val
                85                  90                  95

Val Asp Arg Gly Trp Gly Asn Gly Cys Gly Leu Phe Gly Lys Gly Ser
            100                 105                 110

Ile Asp Thr Cys Ala Lys Phe Ala Cys Ser Thr Lys Ala Ile Gly Arg
        115                 120                 125

Thr Ile Leu Lys Glu Asn Ile Lys Tyr Glu Val Ala Ile Phe Val His
    130                 135                 140

Gly Pro Thr Thr Val Glu Ser His Gly Asn Tyr Ser Thr Gln Ala Gly
145                 150                 155                 160

Ala Thr Gln Ala Gly Arg Phe Ser Ile Thr Pro Ala Ala Pro Ser Tyr
                165                 170                 175

Thr Leu Lys Leu Gly Glu Tyr Gly Glu Val Thr Val Asp Cys Glu Pro
            180                 185                 190

Arg Ser Gly Ile Asp Thr Asn Ala Tyr Tyr Val Met Thr Val Gly Thr
        195                 200                 205

Lys Thr Phe Leu Val His Arg Glu Trp Phe Met Asp Leu Asn Leu Pro
    210                 215                 220

Trp Ser Ser Ala Gly Ser Thr Val Trp Arg Asn Arg Glu Thr Leu Met
225                 230                 235                 240

Glu Phe Glu Glu Pro His Ala Thr Lys Gln Ser Val Ile Ala Leu Gly
                245                 250                 255
```

```
Ser Gln Glu Gly Ala Leu His Gln Ala Leu Ala Gly Ala Ile Pro Val
            260                 265                 270

Glu Phe Ser Ser Asn Thr Val Lys Leu Thr Ser Gly His Leu Lys Cys
        275                 280                 285

Arg Val Lys Met Glu Lys Leu Gln Leu Lys Gly Thr Thr Tyr Gly Val
    290                 295                 300

Cys Ser Lys Ala Phe Lys Phe Leu Gly Thr Pro Ala Asp Thr Gly His
305                 310                 315                 320

Gly Thr Val Val Leu Glu Leu Gln Tyr Thr Gly Thr Asp Gly Pro Cys
                325                 330                 335

Lys Val Pro Ile Ser Ser Val Ala Ser Leu Asn Asp Leu Thr Pro Val
            340                 345                 350

Gly Arg Leu Val Thr Val Asn Pro Phe Val Ser Val Ala Thr Ala Asn
        355                 360                 365

Ala Lys Val Leu Ile Glu Leu Glu Pro Pro Phe Gly Asp Ser Tyr Ile
    370                 375                 380

Val Val Gly Arg Gly Glu Gln Gln Ile Asn His His Trp His Lys Ser
385                 390                 395                 400

Gly Ser Ser Ile Gly Lys Ala Phe Thr Thr Thr Leu Lys Gly Ala Gln
                405                 410                 415

Arg Leu Ala Ala Leu Gly Asp Thr Ala Trp Asp Phe Gly Ser Val Gly
            420                 425                 430

Gly Val Phe Thr Ser Val Gly Lys Ala Val His Gln Val Phe Gly Gly
        435                 440                 445

Ala Phe Arg Ser Leu Phe Gly Gly Met Ser Trp Ile Thr Gln Gly Leu
    450                 455                 460

Leu Gly Ala Leu Leu Leu Trp Met Gly Ile Asn Ala Arg Asp Arg Ser
465                 470                 475                 480

Ile Ala Leu Thr Phe Leu Ala Val Gly Gly Val Leu Leu Phe Leu Ser
                485                 490                 495

Val Asn Val His Ala
            500
```

<210> 21
<211> 676

<212> PRT
<213> Cote d'Ivoire ebolavirus

<400> 21

```
Met Gly Ala Ser Gly Ile Leu Gln Leu Pro Arg Glu Arg Phe Arg Lys
1               5                   10                  15

Thr Ser Phe Phe Val Trp Val Ile Ile Leu Phe His Lys Val Phe Ser
            20                  25                  30

Ile Pro Leu Gly Val Val His Asn Asn Thr Leu Gln Val Ser Asp Ile
        35                  40                  45

Asp Lys Phe Val Cys Arg Asp Lys Leu Ser Ser Thr Ser Gln Leu Lys
    50                  55                  60

Ser Val Gly Leu Asn Leu Glu Gly Asn Gly Val Ala Thr Asp Val Pro
65                  70                  75                  80

Thr Ala Thr Lys Arg Trp Gly Phe Arg Ala Gly Val Pro Pro Lys Val
                85                  90                  95

Val Asn Cys Glu Ala Gly Glu Trp Ala Glu Asn Cys Tyr Asn Leu Ala
            100                 105                 110

Ile Lys Lys Val Asp Gly Ser Glu Cys Leu Pro Glu Ala Pro Glu Gly
        115                 120                 125

Val Arg Asp Phe Pro Arg Cys Arg Tyr Val His Lys Val Ser Gly Thr
    130                 135                 140

Gly Pro Cys Pro Gly Gly Leu Ala Phe His Lys Glu Gly Ala Phe Phe
145                 150                 155                 160

Leu Tyr Asp Arg Leu Ala Ser Thr Ile Ile Tyr Arg Gly Thr Thr Phe
                165                 170                 175

Ala Glu Gly Val Ile Ala Phe Leu Ile Leu Pro Lys Ala Arg Lys Asp
            180                 185                 190

Phe Phe Gln Ser Pro Pro Leu His Glu Pro Ala Asn Met Thr Thr Asp
        195                 200                 205

Pro Ser Ser Tyr Tyr His Thr Thr Thr Ile Asn Tyr Val Val Asp Asn
    210                 215                 220

Phe Gly Thr Asn Thr Thr Glu Phe Leu Phe Gln Val Asp His Leu Thr
225                 230                 235                 240

Tyr Val Gln Leu Glu Ala Arg Phe Thr Pro Gln Phe Leu Val Leu Leu
                245                 250                 255
```

```
Asn Glu Thr Ile Tyr Ser Asp Asn Arg Arg Ser Asn Thr Thr Gly Lys
            260                 265                 270

Leu Ile Trp Lys Ile Asn Pro Thr Val Asp Thr Ser Met Gly Glu Trp
        275                 280                 285

Ala Phe Trp Glu Asn Lys Lys Asn Phe Thr Lys Thr Leu Ser Ser Glu
    290                 295                 300

Glu Leu Ser Phe Val Pro Val Pro Glu Thr Gln Asn Gln Val Leu Asp
305                 310                 315                 320

Thr Thr Ala Thr Val Ser Pro Pro Ile Ser Ala His Asn His Ala Ala
                325                 330                 335

Glu Asp His Lys Glu Leu Val Ser Glu Asp Ser Thr Pro Val Val Gln
            340                 345                 350

Met Gln Asn Ile Lys Gly Lys Asp Thr Met Pro Thr Thr Val Thr Gly
        355                 360                 365

Val Pro Thr Thr Thr Pro Ser Pro Phe Pro Ile Asn Ala Arg Asn Thr
    370                 375                 380

Asp His Thr Lys Ser Phe Ile Gly Leu Glu Gly Pro Gln Glu Asp His
385                 390                 395                 400

Ser Thr Thr Gln Pro Ala Lys Thr Thr Ser Gln Pro Thr Asn Ser Thr
                405                 410                 415

Glu Ser Thr Thr Leu Asn Pro Thr Ser Glu Pro Ser Ser Arg Gly Thr
            420                 425                 430

Gly Pro Ser Ser Pro Thr Val Pro Asn Thr Thr Glu Ser His Ala Glu
        435                 440                 445

Leu Gly Lys Thr Thr Pro Thr Thr Leu Pro Glu Gln His Thr Ala Ala
    450                 455                 460

Ser Ala Ile Pro Arg Ala Val His Pro Asp Glu Leu Ser Gly Pro Gly
465                 470                 475                 480

Phe Leu Thr Asn Thr Ile Arg Gly Val Thr Asn Leu Leu Thr Gly Ser
                485                 490                 495

Arg Arg Lys Arg Arg Asp Val Thr Pro Asn Thr Gln Pro Lys Cys Asn
            500                 505                 510

Pro Asn Leu His Tyr Trp Thr Ala Leu Asp Glu Gly Ala Ala Ile Gly
        515                 520                 525
```

```
Leu Ala Trp Ile Pro Tyr Phe Gly Pro Ala Ala Glu Gly Ile Tyr Thr
    530                 535                 540

Glu Gly Ile Met Glu Asn Gln Asn Gly Leu Ile Cys Gly Leu Arg Gln
545                 550                 555                 560

Leu Ala Asn Glu Thr Thr Gln Ala Leu Gln Leu Phe Leu Arg Ala Thr
                565                 570                 575

Thr Glu Leu Arg Thr Phe Ser Ile Leu Asn Arg Lys Ala Ile Asp Phe
            580                 585                 590

Leu Leu Gln Arg Trp Gly Gly Thr Cys His Ile Leu Gly Pro Asp Cys
        595                 600                 605

Cys Ile Glu Pro Gln Asp Trp Thr Lys Asn Ile Thr Asp Lys Ile Asp
    610                 615                 620

Gln Ile Ile His Asp Phe Val Asp Asn Asn Leu Pro Asn Gln Asn Asp
625                 630                 635                 640

Gly Ser Asn Trp Trp Thr Gly Trp Lys Gln Trp Val Pro Ala Gly Ile
                645                 650                 655

Gly Ile Thr Gly Val Ile Ile Ala Ile Ile Ala Leu Leu Cys Ile Cys
            660                 665                 670

Lys Phe Met Leu
        675
```

<210> 22
<211> 330
<212> PRT
<213> Homo sapiens

<400> 22

```
Ala Ser Phe Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80
```

```
Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330
```

<210> 23
<211> 327
<212> PRT
<213> Homo sapiens

<400> 23

Ala Ser Phe Lys Gly Pro Ser Val Phe Pro Leu Val Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Cys Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Arg Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asp Asn Tyr Lys

260 265 270

Thr Thr Pro Pro Val Leu Asp Ser Asp Gly Ser Phe Phe Leu Tyr Ser
275 280 285

Arg Leu Thr Val Asp Lys Ser Arg Trp Gln Glu Gly Asn Val Phe Ser
290 295 300

Cys Ser Val Met His Glu Ala Leu His Asn His Tyr Thr Gln Lys Ser
305 310 315 320

Leu Ser Leu Ser Pro Gly Lys
325

<210> 24
<211> 97
<212> PRT
<213> Homo sapiens

<400> 24

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Ser Tyr
20 25 30

Ala Met Ser Trp Val Arg Gln Ser Pro Gly Lys Gly Leu Gln Trp Val
35 40 45

Ser Ala Ile Ser Gly Ser Gly Ile Ser Thr Tyr Tyr Ala Asp Ser Val
50 55 60

Arg Gly Arg Phe Thr Ile Ser Arg Asp Asn Ser Lys Asn Thr Leu Tyr
65 70 75 80

Leu Gln Met Ser Ser Leu Ser Arg Gly His Gly Arg Ile Leu Leu Cys
85 90 95

Glu

<210> 25
<211> 214
<212> PRT
<213> Homo sapiens

<400> 25

Val Ile Trp Met Thr Gln Ser Pro Ser Leu Leu Ser Ala Ser Thr Gly
1 5 10 15

Asp Arg Val Thr Ile Ser Cys Arg Met Ser Gln Gly Ile Ser Asn Tyr
20 25 30

```
Leu Ala Trp Tyr Gln Gln Lys Pro Gly Lys Ala Pro Asp Leu Leu Ile
        35                  40                  45

Tyr Ala Ala Ser Thr Leu Gln Ser Gly Val Pro Ser Arg Phe Ser Gly
    50                  55                  60

Ser Gly Ser Gly Thr Asp Phe Ile Leu Thr Ile Ser Arg Leu Gln Ser
65                  70                  75                  80

Glu Asp Phe Ala Ile Tyr Tyr Cys Gln Gln Tyr Tyr Ser Phe Pro Phe
                85                  90                  95

Thr Phe Gly Pro Gly Thr Lys Val Asp Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210
```

<210> 26
<211> 1404
<212> DNA
<213> artificial sequence

<220>
<223> CDS coding for a fusion protein between EPO and GFP

<400> 26

```
atgaaggtcg ctaccacgct aactctcgct tttatctgct gcgcatctgc gtttgggtta      60
aatggccaaa ctactagcgt catgaagaag gtcggattcg gcgccggaag caagccgatg     120
gtgcaggcaa tcgatgttca aggcaaccgt cttggctcca acgctccccc acgcctcatc     180
tgcgacagtc gagttctgga gaggtacatc ttagaggcca aggaggcaga aaatgtcacg     240
atgggttgtg cagaaggtcc cagactgagt gaaaatatta cagtcccaga taccaaagtc     300
```

aacttctatg cttggaaaag aatggaggtg gaagaacagg ccatagaagt ttggcaaggc 360
ctgtccctgc tctcagaagc catcctgcag gcccaggccc tgctagccaa ttcctcccag 420
ccaccagaga cccttcagct tcatatagac aaagccatca gtggtctacg tagcctcact 480
tcactgcttc gggtactggg agctcagaag gaattgatgt cgcctccaga taccacccca 540
cctgctccac tccgaacact cacagtggat actttctgca agctcttccg ggtctacgcc 600
aacttcctcc gggggaaact gaagctgtac acgggagagg tctgcaggag aggggacagg 660
ctggaagttc tgttccaggg gcccatggtg agcaagggcg aggagctgtt caccggggtg 720
gtgcccatcc tggtcgagct ggacggcgac gtaaacggcc acaagttcag cgtgtccggc 780
gagggcgagg gcgatgccac ctacggcaag ctgaccctga agttcatctg caccaccggc 840
aagctgcccg tgccctggcc caccctcgtg accaccttga cctacggcgt gcagtgcttc 900
gcccgctacc ccgaccacat gaagcagcac gacttcttca agtccgccat gcccgaaggc 960
tacgtccagg agcgcaccat cttcttcaag gacgacggca actacaagac ccgcgccgag 1020
gtgaagttcg agggcgacac cctggtgaac cgcatcgagc tgaagggcat cgacttcaag 1080
gaggacggca acatcctggg gcacaagctg gagtacaact acaacagcca caaggtctat 1140
atcaccgccg acaagcagaa gaacggcatc aaggtgaact tcaagacccg ccacaacatc 1200
gaggacggca gcgtgcagct cgccgaccac taccagcaga acacccccat cggcgacggc 1260
cccgtgctgc tgcccgacaa ccactacctg agcacccagt ccgccctgag caaagacccc 1320
aacgagaagc gcgatcacat ggtcctgctg gagttcgtga ccgccgccgg gatcactctc 1380
ggcatggacg agctgtacaa gtaa 1404

<210> 27
<211> 1422
<212> DNA
<213> artificial sequence

<220>
<223> CDS coding for a fusion protein EPO-GFP-HisTag

<400> 27
atgaaggtcg ctaccacgct aactctcgct tttatctgct gcgcatctgc gtttgggtta 60
aatggccaaa ctactagcgt catgaagaag gtcggattcg gcgccggaag caagccgatg 120
gtgcaggcaa tcgatgttca aggcaaccgt cttggctcca acgctccccc acgcctcatc 180
tgcgacagtc gagttctgga gaggtacatc ttagaggcca aggaggcaga aaatgtcacg 240
atgggttgtg cagaaggtcc cagactgagt gaaaatatta cagtcccaga taccaaagtc 300
aacttctatg cttggaaaag aatggaggtg gaagaacagg ccatagaagt ttggcaaggc 360
ctgtccctgc tctcagaagc catcctgcag gcccaggccc tgctagccaa ttcctcccag 420
ccaccagaga cccttcagct tcatatagac aaagccatca gtggtctacg tagcctcact 480
tcactgcttc gggtactggg agctcagaag gaattgatgt cgcctccaga taccacccca 540
cctgctccac tccgaacact cacagtggat actttctgca agctcttccg ggtctacgcc 600

aacttcctcc gggggaaact gaagctgtac acgggagagg tctgcaggag aggggacagg 660

ctggaagttc tgttccaggg gcccatggtg agcaagggcg aggagctgtt caccggggtg 720

gtgcccatcc tggtcgagct ggacggcgac gtaaacggcc acaagttcag cgtgtccggc 780

gagggcgagg gcgatgccac ctacggcaag ctgaccctga agttcatctg caccaccggc 840

aagctgcccg tgccctggcc caccctcgtg accaccttga cctacggcgt gcagtgcttc 900

gcccgctacc ccgaccacat gaagcagcac gacttcttca agtccgccat gcccgaaggc 960

tacgtccagg agcgcaccat cttcttcaag gacgacggca actacaagac ccgcgccgag 1020

gtgaagttcg agggcgacac cctggtgaac cgcatcgagc tgaagggcat cgacttcaag 1080

gaggacggca acatcctggg gcacaagctg gagtacaact acaacagcca caaggtctat 1140

atcaccgccg acaagcagaa gaacggcatc aaggtgaact tcaagacccg ccacaacatc 1200

gaggacggca gcgtgcagct cgccgaccac taccagcaga acacccccat cggcgacggc 1260

cccgtgctgc tgcccgacaa ccactacctg agcacccagt ccgccctgag caaagacccc 1320

aacgagaagc gcgatcacat ggtcctgctg gagttcgtga ccgccgccgg gatcactctc 1380

ggcatggacg agctgtacaa gcaccaccat caccaccatt aa 1422

<210> 28
<211> 20
<212> DNA
<213> artificial sequence

<220>
<223> PCR primer

<400> 28
gtctatatga agctgaaggg 20

<210> 29
<211> 19
<212> DNA
<213> artificial sequence

<220>
<223> PCR primer

<400> 29
gtgagcaagg gcgaggagc 19

<210> 30
<211> 1659
<212> DNA
<213> artificial sequence

<220>
<223> CDS coding for a chimeric protein containing the bipartite topogenic signal sequence fused in-frame with the human GBA

<400> 30
atgagatcct tttgcatcgc agcccttttt gctgtggcat ctgccttcac cacacagcca 60

acttccttca ctgtgaagac tgcgaatgtg ggcgaacggg cgagtggggt tttccctgag 120

cagagctctg ctcatcgcac gcgtaaagca acgattgtca tggatgcccg cccctgcatc 180

```
cctaaaagct tcggctacag ctcggtggtg tgtgtctgca atgccacata ctgtgactcc     240
tttgaccccc cgacctttcc tgcccttggt accttcagcc gctatgagag tacacgcagt     300
gggcgacgga tggagctgag tatggggccc atccaggcta atcacacggg cacaggcctg     360
ctactgaccc tgcagccaga acagaagttc cagaaagtga agggatttgg aggggccatg     420
acagatgctg ctgctctcaa catccttgcc ctgtcacccc ctgcccaaaa tttgctactt     480
aaatcgtact tctctgaaga aggaatcgga tataacatca tccgggtacc catggccagc     540
tgtgacttct ccatccgcac ctacacctat gcagacaccc ctgatgattt ccagttgcac     600
aacttcagcc tcccagagga agataccaag ctcaagatac ccctgattca ccgagccctg     660
cagttggccc agcgtcccgt ttcactcctt gccagcccct ggacatcacc cacttggctc     720
aagaccaatg gagcggtgaa tgggaagggg tcactcaagg gacagcccgg agacatctac     780
caccagacct gggccagata ctttgtgaag ttcctggatg cctatgctga gcacaagtta     840
cagttctggg cagtgacagc tgaaaatgag ccttctgctg ggctgttgag tggataccccc    900
ttccagtgcc tgggcttcac ccctgaacat cagcgagact tcattgcccg tgacctaggt     960
cctaccctcg ccaacagtac tcaccacaat gtccgcctac tcatgctgga tgaccaacgc    1020
ttgctgctgc cccactgggc aaaggtggta ctgacagacc cagaagcagc taaatatgtt    1080
catggcattg ctgtacattg gtacctggac tttctggctc cagccaaagc caccctaggg    1140
gagacacacc gcctgttccc caacaccatg ctctttgcct cagaggcctg tgtgggctcc    1200
aagttctggg agcagagtgt gcggctaggc tcctgggatc gagggatgca gtacagccac    1260
agcatcatca cgaacctcct gtaccatgtg gtcggctgga ccgactggaa ccttgccctg    1320
aaccccgaag gaggacccaa ttgggtgcgt aactttgtcg acagtcccat cattgtagac    1380
atcaccaagg acacgtttta caaacagccc atgttctacc accttggcca cttcagcaag    1440
ttcattcctg agggctccca gagagtgggg ctggttgcca gtcagaagaa cgacctggac    1500
gcagtggcac tgatgcatcc cgatggctct gctgttgtgg tcgtgctaaa ccgctcctct    1560
aaggatgtgc ctcttaccat caaggatcct gctgtgggct tcctggagac aatctcacct    1620
ggctactcca ttcacaccta cctgtggcat cgccagtga                           1659
```

```
<210>  31
<211>  1677
<212>  DNA
<213>  artificial sequence

<220>
<223>  CDS coding for a chimeric GBA-HisTag protein

<400>  31
atgagatcct tttgcatcgc agcccttttt gctgtggcat ctgccttcac cacacagcca      60
acttccttca ctgtgaagac tgcgaatgtg ggcgaacggg cgagtggggt tttccctgag     120
cagagctctg ctcatcgcac gcgtaaagca acgattgtca tggatgcccg cccctgcatc     180
cctaaaagct tcggctacag ctcggtggtg tgtgtctgca atgccacata ctgtgactcc     240
```

tttgaccccc cgacctttcc tgcccttggt accttcagcc gctatgagag tacacgcagt 300
gggcgacgga tggagctgag tatggggccc atccaggcta atcacacggg cacaggcctg 360
ctactgaccc tgcagccaga acagaagttc cagaaagtga agggatttgg aggggccatg 420
acagatgctg ctgctctcaa catccttgcc ctgtcacccc ctgcccaaaa tttgctactt 480
aaatcgtact tctctgaaga aggaatcgga tataacatca tccgggtacc catggccagc 540
tgtgacttct ccatccgcac ctacacctat gcagacaccc ctgatgattt ccagttgcac 600
aacttcagcc tcccagagga agataccaag ctcaagatac ccctgattca ccgagccctg 660
cagttggccc agcgtcccgt ttcactcctt gccagcccct ggacatcacc cacttggctc 720
aagaccaatg gagcggtgaa tgggaagggg tcactcaagg gacagcccgg agacatctac 780
caccagacct gggccagata ctttgtgaag ttcctggatg cctatgctga gcacaagtta 840
cagttctggg cagtgacagc tgaaaatgag ccttctgctg ggctgttgag tggatacccc 900
ttccagtgcc tgggcttcac ccctgaacat cagcgagact tcattgcccg tgacctaggt 960
cctaccctcg ccaacagtac tcaccacaat gtccgcctac tcatgctgga tgaccaacgc 1020
ttgctgctgc cccactgggc aaaggtggta ctgacagacc cagaagcagc taaatatgtt 1080
catggcattg ctgtacattg gtacctggac tttctggctc cagccaaagc caccctaggg 1140
gagacacacc gcctgttccc caacaccatg ctctttgcct cagaggcctg tgtgggctcc 1200
aagttctggg agcagagtgt gcggctaggc tcctgggatc gagggatgca gtacagccac 1260
agcatcatca cgaacctcct gtaccatgtg gtcggctgga ccgactggaa ccttgccctg 1320
aaccccgaag gaggacccaa ttgggtgcgt aactttgtcg acagtcccat cattgtagac 1380
atcaccaagg acacgtttta caaacagccc atgttctacc accttggcca cttcagcaag 1440
ttcattcctg agggctccca gagagtgggg ctggttgcca gtcagaagaa cgacctggac 1500
gcagtggcac tgatgcatcc cgatggctct gctgttgtgg tcgtgctaaa ccgctcctct 1560
aaggatgtgc ctcttaccat caaggatcct gctgtgggct tcctggagac aatctcacct 1620
ggctactcca ttcacaccta cctgtggcat cgccagcacc accatcacca ccattga 1677

<210> 32
<211> 19
<212> DNA
<213> artificial sequence

<220>
<223> PCR primer

<400> 32
ataccaagct caagatacc 19

<210> 33
<211> 20
<212> DNA
<213> artificial sequence

<220>

<223> PCR primer

<400> 33
aactgtaact tgtgctcagc 20

<210> 34
<211> 1605
<212> DNA
<213> artificial sequence

<220>
<223> CDS coding for a chimeric protein containing the bipartite topogenic signal sequence fused in-frame with gp120 coding sequence containing a stop codon

<400> 34
atgagatcct tttgcatcgc agcccttttt gctgtggcat ctgccttcac cacacagcca 60

acttccttca ctgtgaagac tgcgaatgtg ggcgaacggg cgagtggggt tttccctgag 120

cagagctctg ctcatcgcac gcgtaaagca acgattgtca tggataaatt gtgggtcaca 180

gtctattatg gggtacctgt gtggaaggaa gcaaccacca ctctattttg tgcatcagat 240

gctaaagcat atgatacaga ggtacataat gtttgggcca cacatgcctg tgtacccaca 300

gaccccaacc cacaagaagt agtattggta aatgtgacag aaaattttaa catgtggaaa 360

aatgacatgg tagaacagat gcatgaggat ataatcagtt tatgggatca aagcctaaag 420

ccatgtgtaa aattaacccc actctgtgtt agtttaaagt gcactgattt gaagaatgat 480

actaatacca atagtagtag cgggagaatg ataatggaga aaggagagat aaaaaactgc 540

tctttcaata tcagcacaag cataagaggt aaggtgcaga aagaatatgc atttttttat 600

aaacttgata taataccaat agataatgat actaccagct ataagttgac aagttgtaac 660

acctcagtca ttacacaggc ctgtccaaag gtatcctttg agccaattcc catacattat 720

tgtgccccgg ctggttttgc gattctaaaa tgtaataata agacgttcaa tggaacagga 780

ccatgtacaa atgtcagcac agtacaatgt acacatggaa ttaggccagt agtatcaact 840

caactgctgt taaatggcag tctagcagaa gaagaggtag taattagatc tgtcaatttc 900

acggacaatg ctaaaaccat aatagtacag ctgaacacat ctgtagaaat taattgtaca 960

agacccaaca acaatacaag aaaaagaatc cgtatccaga gaggaccagg gagagcattt 1020

gttacaatag gaaaaatagg aaatatgaga caagcacatt gtaacattag tagagcaaaa 1080

tggaataaca ctttaaaaca gatagctagc aaattaagag aacaatttgg aaataataaa 1140

acaataatct ttaagcaatc ctcaggaggg gacccagaaa ttgtaacgca cagttttaat 1200

tgtggagggg aatttttcta ctgtaattca acacaactgt ttaatagtac ttggtttaat 1260

agtacttgga gtactgaagg gtcaaataac actgaaggaa gtgacacaat caccctccca 1320

tgcagaataa aacaaattat aaacatgtgg cagaaagtag gaaaagcaat gtatgcccct 1380

cccatcagtg gacaaattag atgttcatca aatattacag ggctgctatt aacaagagat 1440

ggtggtaata gcaacaatga gtccgagatc ttcagacctg gaggaggaga tatgagggac 1500

aattggagaa gtgaattata taaatataaa gtagtaaaaa ttgaaccatt aggagtagca 1560

cccaccaagg caaagagaag agtggtgcag agagaaaaaa gatga 1605

<210> 35
<211> 1623
<212> DNA
<213> artificial sequence

<220>
<223> CDS coding for a chimeric gp120-HisTag protein

<400> 35
atgagatcct tttgcatcgc agccctttttt gctgtggcat ctgccttcac cacacagcca 60
acttccttca ctgtgaagac tgcgaatgtg ggcgaacggg cgagtggggt tttccctgag 120
cagagctctg ctcatcgcac gcgtaaagca acgattgtca tggataaatt gtgggtcaca 180
gtctattatg gggtacctgt gtggaaggaa gcaaccacca ctctattttg tgcatcagat 240
gctaaagcat atgatacaga ggtacataat gtttgggcca cacatgcctg tgtacccaca 300
gaccccaacc cacaagaagt agtattggta aatgtgacag aaaattttaa catgtggaaa 360
aatgacatgg tagaacagat gcatgaggat ataatcagtt tatgggatca aagcctaaag 420
ccatgtgtaa aattaacccc actctgtgtt agtttaaagt gcactgattt gaagaatgat 480
actaatacca atagtagtag cgggagaatg ataatggaga aaggagagat aaaaaactgc 540
tctttcaata tcagcacaag cataagaggt aaggtgcaga aagaatatgc atttttttat 600
aaacttgata taataccaat agataatgat actaccagct ataagttgac aagttgtaac 660
acctcagtca ttacacaggc ctgtccaaag gtatcctttg agccaattcc catacattat 720
tgtgccccgg ctggttttgc gattctaaaa tgtaataata agacgttcaa tggaacagga 780
ccatgtacaa atgtcagcac agtacaatgt acacatggaa ttaggccagt agtatcaact 840
caactgctgt taaatggcag tctagcagaa gaagaggtag taattagatc tgtcaatttc 900
acggacaatg ctaaaaccat aatagtacag ctgaacacat ctgtagaaat taattgtaca 960
agacccaaca acaatacaag aaaaagaatc cgtatccaga gaggaccagg gagagcattt 1020
gttacaatag gaaaaatagg aaatatgaga caagcacatt gtaacattag tagagcaaaa 1080
tggaataaca ctttaaaaca gatagctagc aaattaagag aacaatttgg aaataataaa 1140
acaataatct ttaagcaatc ctcaggaggg gacccagaaa ttgtaacgca cagttttaat 1200
tgtggagggg aatttttcta ctgtaattca acacaactgt ttaatagtac ttggtttaat 1260
agtacttgga gtactgaagg gtcaaataac actgaaggaa gtgacacaat caccctccca 1320
tgcagaataa aacaaattat aaacatgtgg cagaaagtag gaaaagcaat gtatgcccct 1380
cccatcagtg gacaaattag atgttcatca aatattacag ggctgctatt aacaagagat 1440
ggtggtaata gcaacaatga gtccgagatc ttcagacctg gaggaggaga tatgagggac 1500
aattggagaa gtgaattata taaatataaa gtagtaaaaa ttgaaccatt aggagtagca 1560
cccaccaagg caaagagaag agtggtgcag agagaaaaaa gacaccacca tcaccaccat 1620
tga 1623

<210> 36
<211> 21
<212> DNA
<213> artificial sequence

<220>
<223> PCR primer

<400> 36
cacctcagtc attacacagg c 21

<210> 37
<211> 19
<212> DNA
<213> artificial sequence

<220>
<223> PCR primer

<400> 37
cctcctgagg attgcttaa 19

<210> 38
<211> 142
<212> DNA
<213> Phaeodactylum tricornutum

<400> 38
gggctgcagg acgcaatgga ggattatcac cgcaaaaatg aacttcgaaa aaaactttcg 60

agcgaccatg gaaaaggagg atcagattca gattacaaca gtggattgct ctggtagcaa 120

atatcttctg ctagattggc tc 142

<210> 39
<211> 245
<212> DNA
<213> Phaeodactylum tricornutum

<400> 39
acataccttc agcgtcgtct tcactgtcac agtcaactga cagtaatcgt tgatccggag 60

agattcaaaa ttcaatctgt ttggacctgg ataagacaca agagcgacat cctgacatga 120

acgccgtaaa cagcaaatcc tggttgaaca cgtatccttt tgggggcctc cgctacgacg 180

ctcgctccag ctggggcttc cttactatac acagcgcgca tatttcacgg ttgccagatg 240

tcaag 245

<210> 40
<211> 835
<212> DNA
<213> cauliflower mosaic virus

<400> 40
agattagcct tttcaatttc agaaagaatg ctaacccaca gatggttaga gaggcttacg 60

cagcaggtct catcaagacg atctacccga gcaataatct ccaggaaatc aaataccttc 120

ccaagaaggt taaagatgca gtcaaaagat tcaggactaa ctgcatcaag aacacagaga 180

```
aagatatatt tctcaagatc agaagtacta ttccagtatg gacgattcaa ggcttgcttc      240
acaaaccaag gcaagtaata gagattggag tctctaaaaa ggtagttccc actgaatcaa      300
aggccatgga gtcaaagatt caaatagagg acctaacaga actcgccgta aagactggcg      360
aacagttcat acagagtctc ttacgactca atgacaagaa gaaaatcttc gtcaacatgg      420
tggagcacga cacacttgtc tactccaaaa atatcaaaga tacagtctca gaagaccaaa      480
gggcaattga gacttttcaa caaagggtaa tatccggaaa cctcctcgga ttccattgcc      540
cagctatctg tcactttatt gtgaagatag tggaaaagga aggtggctcc tacaaatgcc      600
atcattgcga taaaggaaag gccatcgttg aagatgcctc tgccgacagt ggtcccaaag      660
atggaccccc acccacgagg agcatcgtgg aaaaagaaga cgttccaacc acgtcttcaa      720
agcaagtgga ttgatgtgat atctccactg acgtaaggga tgacgcacaa tcccactatc      780
cttcgcaaga cccttcctct atataaggaa gttcatttca tttggagaga acacg           835
```

```
<210>  41
<211>  240
<212>  DNA
<213>  Agrobacterium tumefaciens

<400>  41
acgattgaag gagccactca gccgcgggtt tctggagttt aatgagctaa gcacatacgt       60
cagaaaccat tattgcgcgt tcaaaagtcg cctaaggtca ctatcagcta gcaaatattt      120
cttgtcaaaa atgctccact gacgttccat aaattcccct cggtatccaa ttagagtctc      180
atattcactc tcaatccaaa taatctgcaa tggcaattac cttattcgca acttctttac      240
```

```
<210>  42
<211>  242
<212>  DNA
<213>  Phaeodactylum tricornutum

<400>  42
accttcctta aaaatttaat tttcattagt tgcagtcact ccgctttggt ttcacagtca       60
ggaataacac tagctcgtct tcaccatgga tgccaatctc gcctattcat ggtgtataaa      120
agttcaacat ccaaagctag aacttttgga aagagaaaga atatccgaat agggcacggc      180
gtgccgtatt gttggagtgg actagcagaa agtgaggaag gcacaggatg agttttctcg      240
ag                                                                     242
```

```
<210>  43
<211>  253
<212>  DNA
<213>  Agrobacterium tumefaciens

<400>  43
gatcgttcaa acatttggca ataaagtttc ttaagattga atcctgttgc cggtcttgcg       60
atgattatca tataatttct gttgaattac gttaagcatg taataattaa catgtaatgc      120
atgacgttat ttatgagatg ggtttttatg attagagtcc cgcaattata catttaatac      180
gcgatagaaa acaaaatata gcgcgcaaac taggataaat tatcgcgcgc ggtgtcatct      240
```

```
atgttactag atc                                                         253
```

## Description

### FIELD OF THE INVENTION

The present invention is directed to the use of a transformed microalga for the production of glycosylated proteins harboring a pattern of N-glycosylation with high mannose residues, said glycosylated proteins being targeted and stored in the plastid of said microalga. This invention also encompasses methods of producing said glycosylated proteins.

### BACKGROUND OF THE INVENTION

The increasing demand for recombinant drugs has strongly driven the development of various cellular models used as biomanufacturing platforms. Expression systems based on eukaryotic cells are preferentially used for the production of recombinant proteins requiring post-translational modifications (PTM) for their biological activity and/or stability. N-glycosylation is a major PTM characterized by the attachment of glycans onto certain asparagine residues of proteins. N-glycosylation starts when the protein is co-translationally imported into the Endoplasmic Reticulum (ER) leading to mannose-type N-glycans having from 9 to 5 mannose residues which are highly conserved in eukaryotes. Further processing of N-glycans occurs in the Golgi apparatus through the action of various glycosyltransferases. This step leads to mature N-glycosylated proteins harboring organism-specific complex N-glycan structures. Moreover, for a given glycoprotein, N-glycans are often not homogeneous resulting in a pool of structurally distinct oligosaccharides and therefore various glycoforms of said glycoprotein. Complex glycans and heterogeneity can constitute drawbacks of currently available expression systems for the production of recombinant glycoproteins. The occurrence of various glycoforms can thus compromise batch-to-batch consistency resulting in quality and regulatory issues in the production of recombinant drugs. Organism-specific complex N-glycans can also result in issues when using non-human cells. For example, glycoproteins produced in CHO cells can contain N-glycolylneuraminic acid whereas those produced in murine cells contain galactose-$\alpha$(1,3)-galactose which are respectively potentially and highly immunogenic for human. Similarly, insect- or plant-based expression systems also contain potentially immunogenic epitopes such as $\alpha$(1,3)-fucose (in insect and plant cells) and $\beta$(1,2)-xylose (in plant cells).

The present invention discloses the production of glycoproteins and their storage in the plastid of transformed microalgae from the groups of heterokontophytes, haptophytes or cryptophytes. These groups are unique amongst other microalgae or even photosynthetic eukaryotic organisms as they contain plastids surrounded by four membranes with the outermost membrane being interconnected with the ER membrane and studded with ribosomes. This outermost membrane is commonly named Chloroplast Endoplasmic Reticulum (CER). A pathway has been characterized for the targeting of nuclear-encoded proteins in the plastid of these microalgae. Precursors of those proteins are synthesized with an amino-terminal bipartite targeting signal sequence also called "bipartite topogenic signal" (BTS) sequence composed of a signal peptide followed by a transit peptide preceding the sequence of the mature protein. The first step of trafficking in these microorganisms involves the co-translational transport into the ER lumen via the signal peptide. The mechanism for crossing through the second outermost membrane has not been fully identified. Passage through the two innermost membranes to reach the stroma likely involves the transit peptide and translocators showing similarity with plants (see Bolte et al. (2009) Protein targeting into secondary plastids, Journal of Eukaryotic Microbiology, n°56, pp:9-15 for a review of plastids targeting). The evidences of a transport of nuclear-encoded glycosylated proteins to the plastid are scarce and concern plant plastids surrounded by two membranes. These glycoproteins contain complex N-glycan patterns such as $\beta$(1,2)-xylose and $\alpha$(1,3)-fucose residues typical of those added in the Golgi apparatus. The existing literature does not reveal any data concerning the transport of nuclear-encoded glycoproteins to microalgal plastids surrounded by a CER membrane. Besides, background art does not teach or suggest any method for the production of glycoprotein and their storage in the plastidial compartment.

The inventors have surprisingly discovered that the use of a bipartite topogenic signal sequence in a transformed microalga having a CER enabled the expression and glycosylation of proteins in the Endoplasmic Reticulum followed by a transport into the plastid of said microalga without any passage through the Golgi apparatus. N-linked glycans of these glycoproteins are high mannose oligosaccharides (Man-5 to Man-9) characteristic of the ER glycosylation pattern and consequently do not present immunogenic patterns of glycosylation such as those added by glycosyl transferases into the Golgi apparatus. Therefore, the present invention offers an effective method for the production of therapeutic recombinant proteins requiring high mannose glycans for their biological activity without having to inactivate the glycosylation pathway in the Golgi apparatus. Proteins harboring a "high mannose" pattern of glycosylation hold a strong therapeutic interest in the treatment of various diseases. For example, recombinant lysosomal enzymes used for the treatment of lysosomal storage disorders such as Gaucher's or Fabry's diseases require terminal mannose residues for their uptake by human cells. Such glycosylation pattern cannot be directly obtained by CHO cells used for the commercial production of current enzyme replacement therapies and enzymes produced by this system need further deglycosylation steps. The present invention has also applications for the production of viral envelope proteins. For example, the native glycoprotein gp120 of the HIV envelope spike bears N-linked glycans which are almost entirely oligomannose ($Man_{5-9}GlcNAc_2$). These glycans of gp120 ($Man_{6-9}GlcNAc_2$) are important determinant of antibodies recognition including 2G12, one of the most effective HIV neutralizing antibody. In the context of the viral vaccination design, the present invention thus confers a major advantage over conventional platform such as human cell lines for the production of the envelope glycoproteins bearing high mannose glycans, and used as antigens.

The glycans' homogeneity as opposed to mixture of complex glycan structures obtained in other expression systems also constitutes a major benefit of the present invention in term of product quality and consistency. Furthermore, said method is also effective for the production of a high amount of proteins in a stable environment as said proteins are transported and stored into the plastidial stroma.

## SUMMARY OF THE INVENTION

The present invention describes a microalgal-based expression system for the production of glycoproteins and their storage in the plastid. Microalgae used for this invention are species from the groups of heterokonts, cryptophytes and haptophytes that harbor plastid surrounded by an outermost membrane continuous with the Endoplasmic Reticulum. Glycoproteins expressed by mean of the present invention contain targeting sequence allowing their co-translational import into the ER where they undergo N-glycosylation prior to their transport into the plastidial stroma. The present invention enables the production of glycoproteins having a N-glycosylation pattern composed of "high mannose" oligosaccharides, preferably said N-glycosylation pattern being non-immunogenic.

## BRIEF DESCRIPTION OF DRAWINGS

## DETAILED DESCRIPTION OF THE INVENTION

The invention aims to provide a new system for producing proteins harboring a "high mannose" pattern of glycosylation, said proteins being expressed and glycosylated in the Endoplasmic Reticulum of microalgae and further transported in the plastid of said microalgae without any passage through the Golgi apparatus.

An object of the invention is the use of a transformed microalga for the production of a protein harboring a "high mannose" pattern of glycosylation in the plastid of said transformed microalga, wherein

1) said transformed microalga has a Chloroplast Endoplasmic Reticulum (CER);
2) said microalga is transformed with a nucleic acid sequence operatively linked to a promoter, said nucleic acid sequence encoding an amino acid sequence comprising:

    (i) An amino-terminal bipartite topogenic signal (BTS) sequence composed of at least a signal peptide followed by a transit peptide ; and
    *(ii)* The sequence of said protein.

3) the xylosyltransferases and fucosyltransferases of said microalga have not been inactivated;
4) The N-acetylglycosyltransferase I of said microalga has not been inactivated, preferably the N-acetylglycosyltranferases II, III, IV, V and VI, mannosidase II and glycosyltransferases of said microalga have not been inactivated;

The terms "Chloroplast Endoplasmic Reticulum" or "CER" used herein refer to the outermost membrane of the plastid that is continuous with the Endoplasmic Reticulum membrane to which ribosomes are attached. This CER membrane is only found in plastid interconnected with the Endoplasmic Reticulum and harboring four membranes in heterokonts, cryptophytes and haptophytes. (see Bolte et al. (2009) as disclosed previously, Apt et al. (2002) In vivo characterization of diatom multipartite plastid targeting signals, Journal of Cell Science, n°115, pp:4061-4069).

Therefore, microalgae used herein for the production of a protein harboring a non-immunogenic "high mannose" pattern of glycosylation in the plastid are aquatic photosynthetic microorganisms having a Chloroplast Endoplasmic Reticulum, said microalgae being selected in the group comprising heterokonts, cryptophytes and haptophytes.

In a further embodiment, said microalga of the present invention and having a CER is selected in a group of genus comprising *Phaeodactylum, Nitzschia, Skeletonema, Chaetoceros, Odontella, Amphiprora, Thalassiosira, Nannochloropsis, Emiliania, Pavlova, Isochrysis, Apistonema, Rhodomonas.*

In a further embodiment of the present invention, the microalga having a CER is the diatom *Phaeodactylum tricornutum.*

The term "nucleic acid sequence" used herein refers to DNA sequences (e.g., cDNA or genomic or synthetic DNA) and RNA sequences (e. g., mRNA or synthetic RNA), as well as analogs of DNA or RNA containing non-natural nucleotide analogs, non-native internucleoside bonds, or both. Preferably, said nucleic acid sequence is a DNA sequence. The nucleic acid can be in any topological conformation, such as linear or circular.

"Operatively linked" promoter and terminator refers to a linkage in which the promoter is contiguous with the gene of interest, said gene being also contiguous with the terminator in order to control both the expression and transcriptional termination of said gene.

Examples of promoter that drives expression of a polypeptide in a transformed microalga according to the invention include, but are not restricted to, endogenous nuclear promoters such as the promoter of the fucoxanthin-chlorophyll protein A (pFcpA) (GenBank accession number AF219942 from 1 pb to 142 pb, SEQ ID N°38) and the promoter of the fucoxanthin-chlorophyll protein B gene (pFcpB) (GenBank accession number AF219942 from 848 pb to 1092 pb, SEQ ID N°39) from *Phaeodactylum tricornutum* and exogenous promoters such as the promoter 35S (pCaMV35S) from the cauliflower mosaic virus (GenBank accession number AF502128 from 25 pb to 859 pb, SEQ ID N°40), the promoter of the Nopaline Synthase (pNOS) from *Agrobacterium tumefaciens* (GenBank accession number X01077, SEQ ID N°41).

Examples of terminator enabling the transcription termination of a gene in a transformed microalga according to the invention include, but are not restricted to, endogenous nuclear terminators such as the terminator of the fucoxanthin-chlorophyll protein A (tFcpA) (GenBank accession number AF219942 from 1468 pb to 1709 pb, SEQ ID N°42) from *Phaeodactylum tricornutum* and exogenous terminators such as the terminator of the Nopaline Synthase (tNOS) from *Agrobacterium tumefaciens* (GenBank accession number AF502128 from 2778 pb to 3030 pb, SEQ ID N°43).

Transformation of microalgae can be carried out by conventional methods such as microparticles bombardment, electroporation, glass beads, polyethylene glycol (PEG), silicon carbide whiskers, or use of viruses or agrobacterium (see León and Fernández (2007) Transgenic microalgae as green cell factories, *Landes Bioscience*).

In a preferred embodiment, the transformation of a microalga according to the invention is a nuclear transformation for the integration of a foreign nucleic acid sequence into the nuclear genome of said microalga, wherein said nucleic acid sequence may be introduced via a plasmid, virus sequences, double or single strand DNA, circular or linear DNA.

It is generally desirable to include into each nucleotide sequences used for genetic transformation at least one selectable marker to allow selection of microalgae that have been transformed.

Examples of such markers for the transformation of microalga according to the invention are antibiotic resistant genes such as the bleomycin resistance gene (sh ble) enabling resistance to zeocin, nourseothricin resistance genes (nat or sat-1) enabling resistance to nourseothricin, hygromycin phosphotransferase II gene (hptII) enabling resistance to hygromycin or Aminoglycoside-O-phosphotransferase VIII gene (AphVIII) enabling resistance to paromomycin (see also León and Fernández (2007) as disclosed previously).

Alternatively, complementation of auxotrophic mutant strains of microalgae using genes that enable the reversion to prototrophy offers a selection system without the need of antibiotics. Examples of such complementation system include amino acid auxotrophs.

After transformation of microalgae, transformants producing the desired proteins accumulating into the plastidial stroma are selected by the abovementioned selection methods. Alternatively, analysis of the protein to be produced can also be used as a mean of selection on whole microalgae by one or more conventional methods comprising: fluorimeter or immunocytochemistry by exposing cells to an antibody having a specific affinity for the desired protein. This type of selection can also be carried out on disrupted cells by one or more conventional methods comprising: enzyme-linked immunosorbent *assay* (ELISA), mass spectroscopy such as MALDI-TOF-MS, ESI-MS chromatography or spectrophotometer.

The term "signal peptide" as used herein refers to an amino acid sequence located at the amino terminal end of a polypeptide and which mediates the co-translational transport of said polypeptide across the CER membrane and into the ER lumen where cleavage of the signal peptide finally occurs. This signal peptide is typically 15-30 amino acids long, and presents a 3 domains structure (von Heijne (1990) The signal Peptide, Journal of Membrane Biology, n°115, pp:195-201; Emanuelsson et al. (2007) Locating proteins in the cell using TargetP, SignalP and related tools. Nature Protocols, n°2, pp:953-971), which are as follows:

(i) an N-terminal region (n-region) containing positively charged amino acids, such as Arginine (R), Histidine (H) or Lysine (K);
(ii) a central hydrophobic region (h-region) of at least 6 amino acids containing hydrophobic amino acids such as Alanine (A), Cysteine (C), Glycine (G), Isoleucine (I), Leucine (L), Methionine (M), Phenylalanine (F), Proline (P), Tryptophan (W) or Valine (V); and
(iii) a C-terminal region (c-region) of polar uncharged amino acids such as Asparagine (R), Glutamine (Q), Serine (S), Threonine (T) or Tyrosine (Y). Said C-region often contains a helix-breaking proline or glycine that helps define a cleavage site. Small uncharged residues in positions -3 and -1 (defined as the number of residue before the cleavage site) are usually requires for an efficient cleavage by signal peptidase following the translocation across the endoplasmic reticulum membrane (von Heijne (1990) as disclosed previously; Verner and Schatz (1988) Protein translocation across membranes, Science, n°241, pp:1307-1313).

A person skilled in the art is able to simply identify a signal peptide in an amino acid sequence, for example by using the SignalP 3.0 program (accessible on line at http://www.cbs.dtu.dk/services/SignalP/) which predicts the presence and location of signal peptide cleavage sites in amino acid sequences from different organisms by using two different models: the Neural networks and the Hidden Markov models (Emanuelsson *et al.* (2007) as disclosed previously).

The term "transit peptide" as used herein refers to an amino acid sequence that is contiguous to the amino terminal end of a polypeptide and which is sufficient to mediate the transport of said polypeptide through the 3 innermost membranes of the plastid and into the stroma of the plastid where the transit peptide is then cleaved off.

Transit peptides show a broad heterogeneity in term of structural features. They vary in length between 8-150 amino acids.

Preferably, the transit peptide according to the invention comprises less than 60 amino acids.

Transit peptides targeting proteins into the plastidial stroma comprise an aromatic residue such as phenylalanine, tryptophan, or tyrosine at position +1 of the transit peptide relative to the signal peptide's predicted cleavage site.

Transit peptides also possess a high content of hydroxylated residues as well as an overall positive charge due to the presence of basic, positively charged amino acids such as lysine, arginine or histidine and a low content of acidic, negatively charged residues such as aspartate and glutamate (Jarvis (2008) Targeting of nucleus-encoded proteins to chloroplasts in plants, New Phytologist, n°179, pp:257-285). The overall charge of said transit peptide can be calculated as the number of basic, positively charged residues minus the number of acidic, negatively charged residues.

In a preferred embodiment, said transit peptide comprises at least 10% of hydroxylated residues, more preferably at least 13% of hydroxylated residues.

In a preferred embodiment, the overall charge of said transit peptide is at least +1, more preferably at least +2.

A person skilled in the art is able to simply identify a transit peptide in an amino acid sequence, for example by using the TargetP program (accessible on line at http://www.cbs.dtu.dk/services/TargetP/) or iPSORT program (accessible on line at http://ipsort.hgc.jp/). Alternatively, the Hectar program can also be used for the prediction of the whole bipartite topogenic signal sequence in heterokonts (accessible on line at http://www.sb-roscoff.fr/hectar/).

The term "bipartite topogenic signal" sequence or "BTS" as used herein refers to an amino acid sequence that is contiguous to the amino terminal end of a polypeptide and composed of a signal peptide adjoining a transit peptide. Said BTS sequence leads to the co-translational import in the Endoplasmic Reticulum further followed by the transport of the protein in the plastid of the microalga, said protein according to the invention harboring a non-immunogenic "high mannose" pattern of glycosylation. The cleavage of the signal peptide of the aforementioned bipartite topogenic signal sequence in the Endoplasmic Reticulum leads to the exposure of the transit peptide for further targeting of the protein to be produced in the plastid of the transformed microalga.

The bipartite topogenic signal sequence can be identified by bioinformatic analyses performed on protein sequences of heterokonts, cryptophytes or haptophytes from publicly available databases such as the US Department of Energy Joint Genome Institute (JGI, http://vvww.jgi.doe.gov/). The putative protein sequences are screened for the presence of signal peptides using SignalP 3.0. Based on the program's prediction cleavage site, retained sequences are processed to remove amino acids corresponding to the signal peptides and further screened for the presence of transit peptide using TargetP or iPSORT. The transit peptides identified are checked for their overall net charge, the content of hydroxylated residues and the occurrence of an aromatic amino acids at position +1 as previously described. Bipartite topogenic signal sequences can then be retrieved by multi-steps analysis and used in-frame for the targeting of proteins to be produced.

In a preferred embodiment, the invention relates to the use of a transformed microalga for the production of a protein harboring a non-immunogenic "high mannose" pattern of glycosylation in the plastid of said transformed microalga, wherein

1) said transformed microalga has a Chloroplast Endoplasmic Reticulum (CER);
2) said microalga is transformed with a nucleic acid sequence operatively linked to a promoter , said nucleic acid sequence encoding an amino acid sequence comprising:

   (i) An amino-terminal bipartite topogenic signal (BTS) sequence composed of at least a signal peptide followed by a transit peptide ; and
   *(ii)* The sequence of said protein.

3) the xylosyltransferases and fucosyltransferases of said microalga have not been inactivated;
4) The N-acetylglycosyltransferase I of said microalga has not been inactivated, preferably the N-acetylglycosyltranferases II, III, IV, V and VI, mannosidase II and glycosyltransferases of said microalga have not been inactivated; 5) Said BTS sequence leads to the co-translational import of the protein into the Endoplasmic Reticulum followed by the transport of said protein in the plastid of the microalga, said protein harboring a "high mannose" pattern of glycosylation.

In another preferred embodiment, the bipartite topogenic signal sequence is selected in the group comprising:

(a) the amino acid sequence set forth in SEQ ID N°1 from the Light Harversting Complex Protein 11 LHCP11 of *Guillardia theta;*

(b) the amino acid sequence set forth in SEQ ID N°2 from the chloroplast ATPase Gamma subunit (AtpC) protein of *P. tricornutum;*

(c) the amino acid sequence set forth in SEQ ID N°3 from the triose Phosphate/Phosphate Translocator (Tpt1) protein of *P. tricornutum;*

(d) the amino acid sequence set forth in SEQ ID N°4 from the Fucoxanthin-chlorophyll a-c binding protein D (FcpD) protein of *P. tricornutum;*

(e) the amino acid sequence set forth in SEQ ID N°5 from the Fructose-1,6-bisphophatase (FBPC4) protein of *P. tricornutum;*

(f) the amino acid sequence set forth in SEQ ID N°6 from the Oxygen-evolving Enhancer 1 (OEE1) protein of *P. tricornutum;*

wherein the nucleic acid sequence coding for the bipartite topogenic signal is in-frame with the nucleic acid sequence coding for the recombinant protein to be produced. In a most preferred embodiment, the bipartite topogenic signal sequence is selected in the group comprising:

(a) the amino acid sequence set forth in SEQ ID N°2;

(b) the amino acid sequence set forth in SEQ ID N°3;

(c) the amino acid sequence set forth in SEQ ID N°1;

The term "polypeptide" or "protein" as used herein refers to an amino acid sequence comprising amino acids which are linked by peptide bonds. A polypeptide may be monomeric or polymeric. Furthermore, a polypeptide may comprise a number of different domains each of which has one or more distinct activities.

The term "glycosylated polypeptide/protein" or "glycoprotein" as used herein refers to a protein with N-glycosylation.

The protein to be produced according to the invention is a protein harboring a "high mannose" pattern of glycosylation.

The term "N-glycan" as used herein refers to a N-linked oligosaccharide, e.g., one that is attached by a linkage between the N-acetylglucosamine of said oligosaccharide and an asparagine residue at a site of N-glycosylation.

The term "site of N-glycosylation" refers to the asparagine residues of the consensus sequences Asn-X-Ser/Thr, when X is different than proline and aspartic acid, of a protein.

The expressions "high mannose pattern of N-glycosylation" in reference to a protein or "high mannose N-glycosylated protein" refer to a protein harboring high mannose N-linked oligosaccharides, *i.e*, a protein having on each occupied site of N-glycosylation a glycan composed of 5 to 9 mannose residues and at least one exposed mannose residue (terminal mannose residue)..

Preferably, said protein comprises on each occupied N-glycosylation site a glycan composed of 6 to 9 mannose residues and at least one exposed mannose residue.

The term "occupied site of N-glycosylation" refers to a site of N-glycosylation harboring a N-glycan, i.e. to an asparagine residues of the consensus sequences Asn-X-Ser/Thr, when X is different than proline and aspartic acid, harboring oligosaccharides.

Preferably, the protein to be produced according to the invention has at least 5 to 9 mannoses residues, most preferably from 6 to 9 mannose residues on oligosaccharides located at the level of the asparagine residues of the consensus sequences Asn-X-Ser/Thr, when X is different than proline and aspartic acid, of said protein.

Preliminary information about N-glycan of the recombinant protein can be obtained by affino- and immunoblotting analysis using specific probes such as lectins (ConA from *Canavalia ensiformis;* GNL from *Galanthus nivalis;* HHL from *Hippeastrum hybrid ;* ECA from *Erythrina cristagalli;* SNA from *Sambucus nigra;* MAA from *Maackia amurensis* ...) and specific N-glycan antibodies (anti-β(1,2) -xylose; anti-α(1,3)-fucose; anti-Neu5Gc, anti-Lewis ...). To investigate the detailed N-glycan profile of recombinant protein, N-linked oligosaccharides is released from the protein in a non specific manner using enzymatic digestion or chemical treatment. The resulting mixture of reducing oligosaccharides can be profiled by HPLC and/or mass spectrometry approaches (ESI-MS-MS and MALDI-TOF essentially). These strategies, coupled to exoglycosidase digestion, enable N-glycan identification and quantification (Dolashka et al. (2010) Glycan structures and antiviral effect of the structural subunit RvH2 of Rapana hemocyanin, Carbohydrate Research, n° 345, pp:2361-2367).

In a preferred embodiment, the protein to be produced according to the invention is a protein harboring a non-immunogenic "high mannose" pattern of glycosylation.

The expression "non-immunogenic pattern of glycosylation" in reference to a protein to be produced according to the invention refers to a pattern of glycosylation which does not elicit an immune response in the human body. As an example, immunogenic pattern of glycosylation comprising β(1,2)-xylose, α(1,3)-fucose, N-glycolylneuraminic acid and/or galactose-α(1,3)-galactose on N-glycans may elicit immune response. Said non-immunogenic pattern of glycosylation of the protein according to the invention arises from the absence of transit of said protein through the Golgi apparatus in which immunogenic patterns are usually added by glycosyltransferases.

Preferably, a non-immunogenic pattern of glycosylation refers to a pattern of glycosylation not harboring any α(1,3)-fucose or β(1,2)-xylose on N-glycans, *i.e.* on oligosaccharides located at the level of the asparagine residues of the consensus sequences Asn-X-Ser/Thr, when X is different than proline and aspartic acid, of said protein.

In a preferred embodiment, the protein produced in the plastid of microalga according to the invention presents a homogenous pattern of glycosylation.

The term "homogenous" refers to a pattern of glycosylation comprising a majority of "high mannose" N-glycans.

Adavantageously, a homogenous pattern of glycosylation comprises at least 70%, preferablyat least 80% or at least 90%, and most preferably at least 95% of "high mannose" N-glycans.

In another most preferred embodiment, the protein presenting a homogenous pattern of glycosylation according to the invention does not comprise galactose, sialic acid, fucose and/or xylose on N-glycans.

The determination of the homogeneity of a pattern of glycosylation can be realized by analyzing N-glycans as described previously.

Preferably, the spectrum of released N-glycans from the protein produced according to the invention does not comprise peaks corresponding to oligosaccharides having at least one of the following sugar residues: galactose, sialic acid, fucose, xylose.

In a preferred embodiment, the protein according to the invention is a heterologous protein.

The term "heterologous", with reference to a protein, means an amino acid sequence which does not exist in the corresponding microalga before its transformation. It is intended that the term encompasses proteins that are encoded by wild-type genes, mutated genes, and/or synthetic genes.

In a still preferred embodiment, said heterologous protein which is produced and transported in the plastid of a transformed microalga according to the invention can be a protein used for therapeutic purposes, wherein said protein can be of viral or animal origin. Preferably, said animal polypeptide is of mammalian origin. Most preferably, said mammalian polypeptide is of human origin.

In a preferred embodiment, the protein according to the invention is a protein selected in the group comprising human lysosomal enzymes, viral envelope glycoproteins or viral envelope glycoprotein's fragments, antibodies or antibody's fragments and derivatives thereof.

The term "lysosomal enzyme" refers to hydrolases that are naturally produced by the human body having an enzymatic activity in the lysosome organelle. Said enzyme is responsible for breaking down complex chemicals, macromolecules or other materials contained in the lysosome. Deficiencies of such enzymes are responsible for the accumulation of lysosomal metabolites leading to pathologies known as lysosomal storage disorders. Examples of such deficient enzymes and related diseases include:

- α-fucosidase (Fucosidosis)
- α-galactosidase A (Fabry disease)
- α-L-iduronidase (Hurler syndrome; Mucopolysaccharidosis type I)
- Iduronate-2-sulphatase (Hunter syndrome; Mucopolysaccharidosis type II)
- Arylsulfatase B (Maroteaux-Lamy syndrome; Mucopolysaccharidosis type VI)
- Acid α-mannosidase (α-mannosidosis)
- α-neuraminidase (sialidosis)
- Acid α- glucosidase (Pompe disease)

- Acid β-galactosidase (GM1 gangliosidosis)
- Acid β-glucosidase (Gaucher disease)
- β-glucuronidase (Sly syndrome; MPS VII)
- Acid β-mannosidase (β-mannosidosis)
- Acid Sphingomyelinase (Niemann-Pick disease)
- Lysosomal acid lipase (Wolman disease)

Examples of lysosomal enzymes to be produced using the present invention include α-galactosidase A, α-fucosidase, α-L-iduronidase, iduronate-2-sulfatase, arylsulfatase B, acid sphingomyelinase, acid α-mannosidase, acid α-glucosidase, α-neuraminidase, acid β-galactosidase, acid β-glucosidase, β-glucuronidase, acid β-mannosidase and lysosomal acid lipase.

Lysosomal enzymes produced according to the invention harbor high-mannose oligosaccharides and therefore can be taken up by cells through their mannose receptors to replace deficient enzymes in the so-called enzyme replacement therapy (ERT).

The term "Viral envelope glycoprotein" refers to a glycosylated protein included in the viral envelope covering the protein capsid of the virion particle. Said viral envelope glycoprotein is located on the surface of the envelope enabling the binding of the virion particle onto receptors of host cell leading ultimately to entry of the virus into the cell.

Examples of such viral envelope glycoproteins are the precursor gp160 and its processed forms gp120 and gp41 proteins from type 1 human immunodeficiency virus (HIV), E1 and E2 proteins from hepatitis C virus, the E protein from the dengue virus and west nile virus, the GP protein from Ebola virus.

The term "viral envelope glycoprotein's fragments" as used herein refers to fragments of said envelope glycoprotein.

An "antibody" is an immunoglobulin molecule corresponding to a tetramer comprising four polypeptide chains, two identical heavy (H) chains (about 50-70 kDa when full length) and two identical light (L) chains (about 25 kDa when full length) inter-connected by disulfide bonds. Light chains are classified as kappa and lambda. Heavy chains are classified as gamma, mu, alpha, delta, or epsilon, and define the antibody's isotype as IgG, IgM, IgA, IgD, and IgE, respectively. Each heavy chain is comprised of an amino-terminal heavy chain variable region (abbreviated herein as HCVR) and a heavy chain constant region. The heavy chain constant region is comprised of three domains (CH1, CH2, and CH3) for IgG, IgD, and IgA; and 4 domains (CH1, CH2, CH3, andCH4) for IgM and IgE. Each light chain is comprised of an amino-terminal light chain variable region (abbreviated herein as LCVR) and a light chain constant region. The light chain constant region is comprised of one domain, CL. The HCVR and LCVR regions can be further subdivided into regions of hypervariability, termed complementarity determining regions (CDRs), interspersed with regions that are more conserved, termed framework regions (FR). Each HCVR and LCVR is composed of three CDRs and four FRs, arranged from amino-terminus to carboxy-terminus in the following order: FR1, CDR1, FR2, CDR2, FR3, CDR3, FR4. The assignment of amino acids to each domain is in accordance with well-known conventions. The functional ability of the antibody to bind a particular antigen depends on the variable regions of each light/heavy chain pair, and is largely determined by the CDRs.

The term "antibody", as used herein, refers to a monoclonal antibody *per se.* A monoclonal antibody can be a human antibody, chimeric antibody and/or humanized antibody.

Antibodies to be produced according to the invention are for example recombinant IgG antibodies having enhanced antibody dependent cell-mediated cytotoxicity (ADCC).

The term "antibody fragments" as used herein refers to antibody fragments that bind to the particular antigens of said antibody. For example, antibody fragments capable of binding to particular antigens include Fab (e.g., by papain digestion), Fab' (e.g., by pepsin digestion and partial reduction) and F(ab')2 (e.g., by pepsin digestion), facb (e.g., by plasmin digestion), pFc' (e.g., by pepsin or plasmin digestion), Fd (e.g., by pepsin digestion, partial reduction and reaggregation), Fv or scFv (e.g., by molecular biology techniques) fragments, are encompassed by the invention. Such fragments can be produced by enzymatic cleavage, synthetic or recombinant techniques, as known in the art and/or as described herein. Antibodies can also be produced in a variety of truncated forms using antibody genes in which one or more stop codons have been introduced upstream of the natural stop site. For example, a combination gene encoding a F(ab')2 heavy chain portion can be designed to include DNA sequences encoding the $CH_1$ domain and/or hinge region of the heavy chain. The various portions of antibodies can be joined together chemically by conventional techniques, or can be prepared as a contiguous protein using genetic engineering techniques.

As used herein, the term "derivative" refers to a polypeptide having a percentage of identity of at least 90% with the complete amino acid sequence of any of the protein disclosed previously and having the same activity.

Preferably, a derivative has a percentage of identity of at least 95% with said amino acid sequence, and preferably of at least 99% with said amino acid sequence.

As used herein, "percentage of identity" between two amino acids sequences, means the percentage of identical amino acids, between the two sequences to be compared, obtained with the best alignment of said sequences, this percentage being purely statistical and the differences between these two sequences being randomly spread over the amino acids sequences. As used herein, "best alignment" or "optimal alignment", means the alignment for which the determined percentage of identity (see below) is the highest. Sequences comparison between two amino acids sequences are usually realized by comparing these sequences that have been previously aligned according to the best alignment; this comparison is realized on segments of comparison in order to identify and compare the local regions of similarity. The best sequences alignment to perform comparison can be realized by using computer softwares using algorithms such as GAP, BESTFIT, BLAST P, BLAST N, FASTA, TFASTA in the Wisconsin Genetics software Package, Genetics Computer Group, 575 Science Dr., Madison, WI USA. To get the best local alignment, one can preferably used BLAST software, with the BLOSUM 62 matrix, preferably the PAM 30 matrix. The identity percentage between two sequences of amino acids is determined by comparing these two sequences optimally aligned, the amino acids sequences being able to comprise additions or deletions in respect to the reference sequence in order to get the optimal alignment between these two sequences. The percentage of identity is calculated by determining the number of identical position between these two sequences, and dividing this number by the total number of compared positions, and by multiplying the result obtained by 100 to get the percentage of identity between these two sequences.

In a most preferred embodiment of the invention, proteins to be produced according to the invention are selected in the group comprising the sequences disclosed in Table I or derivatives thereof, wherein said protein sequences are fused downstream of a bipartite topogenic signal peptide.

Table I

| PROTEIN | CDS SEQ ID N° | Accession number (Protein) | Comments |
|---|---|---|---|
| β-glucocerebrosidase = Acid β-glucosidase | SEQ ID N° 7 | AAA35873 | Lysosomal enzyme |
| α-Galactosidase A | SEQ ID N°8 | NP_000160 | Lysosomal enzyme |
| Alglucosidase = Acid α-glucosidase | SEQ ID N°9 | NP_000143 | Lysosomal enzyme |
| α-L-iduronidase | SEQ ID N°10 | NP_000194 | Lysosomal enzyme |
| Iduronate 2-sulfatase | SEQ ID N°11 | NP_000193 | Lysosomal enzyme |
| Arylsulfatase B | SEQ ID N°12 | NP_000037 | Lysosomal enzyme |
| Acid Sphingomyelinase | SEQ ID N°13 | NP_000534 | Lysosomal enzyme |
| Lysosomal acid lipase | SEQ ID N°14 | NP_001121077 | Lysosomal enzyme |
| GP120 | SEQ ID N°15 | NP_579894 | Envelope glycoprotein from Human Immunodeficiency Virus 1 |
| GP41 | SEQ ID N°16 | NP_579895 | Envelope transmembrane glycoprotein from Human Immunodeficiency Virus 1 |
| E1 protein | SEQ ID N°17 | From aa 192 to 383 of the polyprotein P27958 | Envelope glycoprotein from Hepatitis C Virus |
| E2 protein | SEQ ID N°18 | From aa 384 to 746 of the polyprotein P27958 | Envelope glycoprotein from Hepatitis C Virus |
| E protein | SEQ ID N°19 | From aa 281 to 775 of the polyprotein AD097105 | Envelope glycoprotein from Dengue virus 1 |
| E protein | SEQ ID N°20 | From aa 291 to 791 of the polyprotein ADL27981 | Envelope glycoprotein from West Nile Virus |
| Spike glycoprotein precursor | SEQ ID N°21 | ACI28632 | Envelope glycoprotein from Ebola virus |
| immunoglobulin heavy chain constant region gamma | SEQ ID N°22 | CAC20454 | Gamma 1 |
| | SEQ ID N°23 | CAC20457 | Gamma 4 |

(continued)

| PROTEIN | CDS SEQ ID N° | Accession number (Protein) | Comments |
|---|---|---|---|
| Immunoglobulin Variable Heavy Chain | SEQ ID N°24 | AAA59127 | |
| Immunoglobulin Kappa light Chain (VL+CL) | SEQ ID N°25 | CAA09181 | |

Still most preferably, said glycosylated protein is the β-glucocerebrosidase as encoded by the amino acid sequence set forth in SEQ ID N°7, the α-Galactosidase A as encoded by the amino acid sequence set forth in SEQ ID N°8, the α-L-iduronidase as encoded by the amino acid sequence set forth in SEQ ID N°10, the Alglucosidase as encoded by the amino acid sequence set forth in SEQ ID N°9, the Acid Sphingomyelinase as encoded by the amino acid sequence set forth in SEQ ID N°13, the GP120 (HIV) as encoded by the amino acid sequence set forth in SEQ ID N°15, the E1 (HCV) as encoded by the amino acid sequence set forth in SEQ ID N°17 and the E2 (HCV) as encoded by the amino acid sequence set forth in SEQ ID N°18.
According to the invention, xylosyltransferases and fucosyltransferases from the microalga used for the production of a protein harboring a non-immunogenic "high mannose" pattern of N-glycosylation have not been inactivated.
The expression "not to have been inactivated" with reference to an enzyme means that the activity of said enzyme in the microalga of the invention has neither been modified nor suppressed by its transformation.
Xylosyltransferases are enzymes having an activity of adding β(1,2)-linked xyloses on N-glycans of glycoproteins in the Golgi apparatus.
Fucosyltransferases are enzymes having an activity of adding α(1,3)-linked fucoses on N-glycans of glycoproteins in the Golgi apparatus.
Still according to the invention, the N-acetylglucosaminyltransferase I has not been inactivated.
The N-acetylglucosaminyltransferase I is capable of adding an N-acetylglucosamine (GlcNac) residue to $Man_5GlcNac_2$ to produce $GlcNacMan_5GlcNac_2$ in the Golgi apparatus.
Preferably, the N-acetylglucosaminyltranferases II, III, IV, V and VI, the mannosidase II and the glycosyltransferases of the glycosylation pathway of said microalga have not been inactivated.
Glycosyltransferases comprise galactosyltransferases, fucosyltransferases, xylosyltransferases and sialyltransferases.
Another object of the invention is a vector comprising a nucleic acid sequence operatively linked to a promoter, wherein said nucleic acid sequence encodes an amino acid sequence comprising:

i) a bipartite topogenic signal (BTS) composed of at least a signal peptide and a transit peptide as defined previously, and
ii) the sequence of a protein to be produced as defined previously.

The term "vector" refers to any vehicle capable of facilitating the transfer of a nucleic acid sequence in a microalga. Said term "vector" encompasses without limitation the plasmids, cosmids, phagemids or any other vehicle derived from viral or proteic sources which have been manipulated for the insertion or incorporation of a nucleic acid sequence into a microalga.
In a preferred embodiment, the vector according to the invention also comprises a nucleic acid sequence encoding a selectable marker operatively linked to a promoter as defined previously. Alternatively, a nucleic acid sequence encoding a protein enabling the restoration of prototrophy operatively linked to a promoter can be included in the vector of the present invention.
Another object of the invention is a microalga comprising a nucleic acid sequence operatively linked to a promoter, wherein said nucleic acid sequence encodes an amino acid sequence comprising:

i) a bipartite topogenic signal (BTS) composed of at least a signal peptide and a transit peptide as defined previously, and
ii) the sequence of a protein as defined previously.

Another embodiment of the invention discloses a microalga comprising a vector as defined previously.
Another object of the invention is a method for producing a protein in a transformed microalga having a Chloroplast Endoplasmic Reticulum (CER) as defined previously, said method comprising the steps of:

1) Culturing said transformed microalga;

2) Harvesting the plastid of said transformed microalga;
3) Purifying said protein from said plastid.

The culture of the transformed microalga according to the invention can be carried out by conventional methods of culture according to the specie of the microalga which has been selected for the transformation and production of proteins. A protocol that can be used for the cultivation of microalgae of the present invention is given in the example section.

Method for isolation of plastid from transformed microalga according to the invention includes, but is not limited to, the use of density gradient centrifugation. Said method includes an initial step to release the microalgal cell content by homogenization in a medium containing sorbitol followed by a purification step on a 40% Percoll continuous gradient. Alternatively, the method of "cell disruption" leading to the release of the whole intracellular content can be used for the releasing of the plastid content. A method for cell disruption of microalgae of the present invention by sonication is given in the example section.

The purification of the protein to be produced according to the invention can be carried out by chromatography. Such method includes the use of filtration followed by concanavalin A chromatography to specifically purified glycoproteins. Gel filtration and ion-exchange chromatography can also be used to purify further the recombinant polypeptide.

In another embodiment, the protein of the invention can be fused to an amino- or carboxy-terminal Tag for the purpose of purification of such protein. The term "Tag" as used herein refers to an amino acid sequence fused to a protein. An example of Tag include the histidine tag composed of six histidine residues that can be purified as described in the example section.

In another embodiment, the method for producing a glycoprotein stored in the plastid of said transformed microalga comprises a former step of transforming said microalga with a nucleic acid sequence operatively linked to a promoter as defined previously.

In another embodiment, the method for producing a glycoprotein stored in the plastid of said transformed microalga comprises a former step of transforming said microalga with a vector as defined previously.

In another embodiment of the invention, the method for producing a protein stored in the plastid of a transformed microalga further comprises a step 4) of determining the N-glycosylation pattern of said protein and selecting the protein harboring a "high mannose" pattern of N-glycosylation .

Preliminary information about N-glycosylation of the recombinant protein accumulated in the plastid can be obtained by affinodetection analysis using specific probes such as lectins (ConA from *Canavalia ensiformis;* GNL from *Galanthus nivalis;* HHL from *Hippeastrum hybrid ;* ECA from *Erythrina cristagalli;* SNA from *Sambucus nigra;* MAA from *Maackia amurensis...*). Lack of β(1,2)-xylose, α(1,3)-fucose, Neu5Gc and Lewis epitopes can be assessed by immunoblotting analysis using specific N-glycans antibodies (antiβ(1,2)-xylose; anti-α(1,3)-fucose; anti-Neu5Gc; anti-Lewis ...). To investigate the detailed N-glycan profile of recombinant polypeptide, N-linked oligosaccharides is released from the polypeptide in a non specific manner using enzymatic digestion or chemical treatment. The resulting mixture of reducing oligosaccharides can be profiled by HPLC and/or mass spectrometry approaches (ESI-MS-MS and MALDI-TOF essentially). These strategies, coupled to exoglycosidase digestion, enable N-glycan identification and quantification (Séveno et al. (2008) Plant N-glycan profiling of minute amounts of material, Analytical Biochemistry, n°379, pp:66-72; Dolashka *et al.* (2010) as disclosed previously).

In a preferred embodiment, the method of producing proteins harboring a "high mannose" N-glycosylation pattern in the plastid of transformed microalgae leads to the transport of at least 70%; preferably 80% and most preferably 90% of said proteins in the plastid.

Said quantity of transported proteins in the stroma of the plastid of said microalgae can be determined using the ratio between the overall quantity of the recombinant protein and the quantity of said protein within the plastid. The overall quantity of recombinant protein is being defined as the sum of intracellular and extracellular quantity of said recombinant protein. Intracellular content of the aforementioned protein can be obtained by cell disruption method while plastidial content of said protein is obtained by purification of said organelle as described previously. Quantities of the recombinant protein can be determined on the intracellular, extracellular and plastidial fractions by enzyme-linked immunosorbent assay (ELISA) on fractions. The percentage of protein stored in the plastid can be calculated as follow:

$$\%_{plastidial} = (Q_{plastidial} \times 100) / (Q_{internal} + Q_{external})$$

Wherein:

$Q_{plastidial}$ is the quantity of the recombinant protein to be produced detected in the fraction of purified plastid.

$Q_{internal}$ is the quantity of the recombinant protein to be produced detected in the intracellular fraction.

$Q_{external}$ is the quantity of the recombinant protein to be produced detected in the extracellular fraction.

$\%_{plastidial}$ is the percentage of the recombinant protein to be produced accumulated within plastid.

Another object of the invention is a protein harboring a non-immunogenic « high mannose » pattern of glycosylation and produced according to the method as defined previously.

Another object of the invention is a pharmaceutical composition comprising a protein harboring a « high mannose » pattern of glycosylation and produced according to the method of the invention.

In the following examples, the invention is described in more detail with reference to methods. Yet, no limitation of the invention is intended by the details of the examples. Rather, the invention pertains to any embodiment which comprises details which are not explicitly mentioned in the examples herein, but which the skilled person finds without undue effort.

## EXAMPLES

### Example 1 Targeting of a nuclear-encoded glycosylated chimeric protein into the plastid of *Phaeodactylum tricornutum*

To test the ability of a nuclear-encoded protein to be glycosylated, targeted and stored into the plastid, *Phaeodactylum tricornutum* (*P. tricornutum*) was transformed with a plasmid containing a 54 amino acids bipartite topogenic signal sequence of the phosphoenolpyruvate/phosphate translocator (Tpt1) from *P. tricornutum* fused in-frame with a sequence coding for a chimeric protein composed of the mature murine erythropoietin (EPO) and the enhanced green fluorescent protein (GFP) (SEQ ID N°26). The chimeric protein is composed of 166 amino acids corresponding to the mature EPO protein lacking its native 26 amino acids signal peptide followed by the PreScission protease cleavage site (LEVLFQGP) and 239 amino acids corresponding to the green fluorescent protein. The chimeric protein obtained contains 3 potential N-glycosylation sites within the EPO sequence.

a) Standard culture conditions of *Phaeodactylum tricornutum*

The diatom *Phaeodactylum tricornutum* was grown at 20°C under continuous illumination (280-350 μmol photons.$m^{-2}.s^{-1}$), in natural coastal seawater sterilized by 0.22 μm filtration. This seawater is enriched with nutritive Conway media with addition of silica (40 $mg.L^{-1}$ of sodium metasilicate). For large volume (from 2 litters to 300 liters), cultures were aerated with a 2% $CO_2$/air mixture to maintain the pH in a range of 7.5-8.1.

For genetic transformation, diatoms were spread on gelose containing 1% of agar. After concentration by centrifugation, the diatoms were spread on petri dishes sealed and incubated at 20°C under constant illumination. Concentration of culture was estimated on Mallassez counting cells after fixation of microalgae with a Lugol's solution.

b) Expression constructs for genetic transformation

The cloning vector pPha-T1 (GenBank accession number AF219942) includes sequences of *P. tricornutum* promoter fcpA (fucoxanthin-chlorophyll a/c-binding proteins A) upstream of a multiple cloning site followed by the terminator fcpA. It also contains a selection cassette with the promoter fcpB (fucoxanthin-chlorophyll a/c-binding proteins B) upstream of the coding sequence sh ble followed by the terminator fcpA (Zaslavskaia and Lippmeier (2000) transformation of the diatom Phaeodactylum tricornutum (Bacillariophyceae) with a variety of selectable marker and reporter genes, Journal of Phycology, n°36,pp379-386). The sequence containing the bipartite topogenic signal sequence fused in-frame with the chimeric EPO-GFP protein (nucleic acid sequence SEQ ID N°26) was synthesized with the addition of EcoRI and HindIII restriction sites flanking the 5' and 3' ends respectively. Alternatively, a similar sequence containing a histidine tag at the carboxy-terminal (EPO-GFP-HisTag) (nucleic acid sequence SEQ ID N°27) was also synthesized with the addition of EcoRI and HindIII restriction sites flanking the 5' and 3' ends respectively. After digestion by EcoRI and HindIII, each insert was introduced into the pPHA-T1 vector. As a control, an empty pPha-T1 vector lacking the EPO-GFP coding sequence was used.

c) Genetic transformation

The transformation was carried out by particles bombardment using the BIORAD PDS-1000/He apparatus modified (Thomas et al. (2001) A helium burst biolistic device adapted to penetrate fragile insect tissues, Jonal of Insect

Science, n°1, pp1-9).

Cultures of diatoms (*P. tricornutum*) in exponential growth phase were concentrated by centrifugation (10 minutes, 2150 g, 20°C), diluted in sterile seawater, and spread on agar plate at $10^8$ cells per plate. The microcarriers are gold particles (diameter 0.6 $\mu$m). Microcarriers were prepared according to the protocol of the supplier (BIORAD). Parameters used for shooting were the following:

- use of the long nozzle,
- use of the stopping ring with the largest hole,
- 15 cm between the stopping ring and the target (diatom cells),
- precipitation of the DNA by a solution containing 1.25 M $CaCl_2$ and 20 mM spermidine,
- a ratio of 1.25 $\mu$g DNA for 0.75 mg gold particles per shot,
- 900 psi rupture disk with a distance of escape of 0.2 cm,
- a vacuum of 30 Hg

Diatoms were incubated 24 hours before the addition of the antibiotic zeocin (100 $\mu g.ml^{-1}$) and were then maintained at 20°C under constant illumination. After 1-2 weeks of incubation, individual clones were picked from the plates and inoculated into liquid medium containing zeocin (100 $\mu g.ml^{-1}$).

d) <u>Microalgae DNA extraction</u>

Cells ($5.10^8$) transformed by the various vectors were pelleted by centrifugation (2150 g, 15 minutes, 4°C). Microalgae cells were incubated overnight at 4°C with 4 mL of TE NaCl 1X buffer (Tris-HCL 0.1 M, EDTA 0.05 M, NaCl 0.1 M, pH 8). 1% SDS, 1% Sarkosyl and 0.4 $mg.mL^{-1}$ of proteinase K were then added to the sample, followed by an incubation at 40°C for 90 minutes. A first phenol-chloroform-isoamyl alcohol extraction was carried out to extract an aqueous phase comprising the nucleic acids. RNA contained in the sample was eliminated by an hour incubation at 60°C in the presence of RNase (1 $\mu g.mL^{-1}$). A second phenol-chloroform extraction was carried out, followed by a precipitation with ethanol. The pellet obtained was air-dried and solubilised into 200$\mu$ L of ultrapure sterile water. Quantification of DNA was carried out by spectrophotometry (260 nm) and analysed by agarose gel electrophoresis.

e) <u>Polymerase chain reaction (PCR) analysis</u>

The incorporation of the heterologous chimeric EPO-GFP sequence in the genome of *Phaeodactylum tricornutum* was assessed by PCR analysis. The sequences of primers used for the PCR amplification were 5'-GTCTATATGAAGCTGAAGGG-3' (SEQ ID N°28) and 5'-GTGAGCAAGGGCGAGGAGC-3' (SEQ ID N°29) located in the EPO and GFP sequence respectively. The PCR reaction was carried out in a final volume of 50 $\mu$l consisting of 1X PCR buffer, 0.2 mM of each dNTP, 5 $\mu$M of each primer, 20 ng of template DNA and 1.25 U of *Taq* DNA polymerase (Taq DNA polymerase, ROCHE). Thirty cycles were performed for the amplification of template DNA. Initial denaturation was performed at 94°C for 4 min. Each subsequent cycle consisted of a 94°C (1 min) melting step, a 55°C (1 min) annealing step, and a 72°C (1 min) extension step. Samples obtained after the PCR reaction were run on agarose gel (1%) stained with ethidium bromide.

Results revealed a single band at 276 bp for cells transformed with the constructs carrying the bipartite topogenic signal sequence fused to the chimeric EPO-GFP (data not shown). No band was detected in cells transformed with the control vector. This result validated the incorporation of the exogenous gene in the genome of *Phaeodactylum tricornutum.*

f) <u>Sub-cellular localization of the chimeric protein</u>

To investigate the sub-cellular localization of the chimeric protein EPO-GFP, confocal microscopy was performed on wild-type and transformed cells of *P. tricornutum.* GFP and chlorophyll fluorescence were excited at 488 nm, filtered and detected by two different photomultiplier tubes with bandwidths of 500-520 and 625-720 nm for GFP and chlorophyll fluorescence, respectively.

Confocal microscopy revealed the co-localization of the GFP signal (data not shown) with the position of the plastid as observed by bright field and autofluorescence of chlorophyll (data not shown) as well as merged images (data not shown). This result revealed that the use of the amino-terminal bipartite topogenic signal sequence from Tpt1 allowed the targeting of the chimeric protein EPO-GFP to the chloroplast of *P. tricornutum.*

g) Immunoblotting analysis

Aliquotes of wild-type and transformed cells of *P. tricornutum* culture at exponential phase of growth are collected and cells are separated from the culture medium by centrifugation (10 minutes, 2150 g, 20°C). Cell pellets are resuspended in Tris-HCl 0.15 M pH 8, saccharose 15%, SDS 0.5%, PMSF 1 mM, protease inhibitor cocktail 1% (SIGMA) and sonicated for 30 min. Cell suspensions obtained are centrifuged (60 minutes, 15000 g, 4°C) to remove cell debris and supernatants correspond to the intracellular fraction.

Ten μL of intracellular fractions from EPO-GFP transformed and wild-type cells are separated by SDS-PAGE using a 12% polyacrylamide gel. The separated proteins are transferred onto nitrocellulose membrane and stained with Ponceau Red in order to control transfer efficiency. The nitrocellulose membrane is blocked overnight in milk 5% dissolved in TBS for immunodetection. Immunodetection is then performed using anti-EPO (R&D SYSTEMS, AF959) (1:500 in TBS-T containing milk 1% for 2h at room temperature) or horseradish peroxidase-conjugated anti-GFP (Santa Cruz, sc-9996-HRP) (1:2000 in TBS-T containing milk 1% for 2h at room temperature). Membrane incubated with the anti-EPO antibody is then washed with TBS-T (6 times, 5 minutes, room temperature) and binding of the primary antibody is revealed upon incubation with a secondary horseradish peroxidase-conjugated rabbit anti-goat IgG (SIGMA-ALDRICH, A8919) (1:10000 in TBS-T containing milk 1% for 1.5h at room temperature). All membranes are then washed with TBS-T (6 times, 5 minutes, room temperature) followed by a final wash with TBS (5 minutes, room temperature). Final development of the blots is performed by chemiluminescence method.Alternatively, deglycosylation assays can be performed on the protein extract prior to immunoblotting experiment using either peptide-*N*-glycosidase F (PNGase F, New England Biolabs) or endoglycosidase H (Endo H, New England Biolabs) according to manufacturer's recommendations.

h) Purification of the chimeric protein

The chimeric protein EPO-GFP carrying the histidine tag is purified by chromatography method. Intracellular fractions from EPO-GFP-HisTag as well as wild-type cells (control) are prepared as previously described. Both fractions are filtered using a membrane filter of 0.22 μm pore size, concentrated 10 times, and buffer-exchanged with 20 mM Tris, pH 9 containing 5 mM imidazole using a concentration device (MILLIPORE, Amicon Ultra-15, 3 kDa). Purification is performed using the AKTA FPLC system (GE Healthcare) and a Ni Sepharose column (GE Healthcare). The column is equilibrated with 20 mM Tris, pH 9.0 buffer containing 5 mM imidazole and the sample is then loaded. The column is washed with buffer containing 10 mM imidazole followed by elution with buffer containing 200 mM imidazole. The peak is collected and loaded on a Sephadex G-50 column equilibrated with 5 mM sodium phosphate buffer, pH 7.4. The desalted protein is collected, concentrated using a concentration device (MILLIPORE, Amicon Ultra-15, 3 kDa) and analysed by immunoblotting.

i) Structural characterization of N-linked glycans of the chimeric protein

The chimeric EPO-GFP carrying the histidine tag purified by chromatography method is subjected to enzymatic deglycosylation using PNGase F or Endo H in order to release N-linked glycans. Released glycans are analyzed by mass spectrometry as described by Dolashka et al. (2010) Glycan structures and antiviral effect of the structural subunit RvH2 of Rapana hemocyanin, Carbohydr Res, 345:2361-2367.

### Example 2 Targeting of nuclear-encoded human lysosomal enzyme into the plastid of *Phaeodactylum tricornutum*

The β-glucocerebrosidase (GBA) is an enzyme naturally targeted to the lysosomal compartments of human cells. Enzymatic deficiency leads to the accumulation of glucocerebroside in macrophages causing Gaucher's disease. Treatments include enzyme replacement therapy based on the delivery of intravenously injected recombinant β-glucocerebrosidase. The FDA-approved drug is produced in Chinese Hamster Ovary cells and modified by sequential deglycosylation of its carbohydrate side chains to expose alpha-mannosyl residues that mediate uptake of the therapeutic enzyme by surface mannose receptor expressed on target cells. Consequently, there is an industrial benefit to produce a recombinant β-glucocerebrosidase having naturally N-glycans with mannose-terminated structures.

Human β-glucocerebrosidase is expressed, targeted and stored into the plastidial stroma of *P. tricornutum* by means of the present invention. A plasmid containing a 55 amino acids bipartite topogenic signal sequence of the ATPase gamma subunit (atpC) from *P. tricornutum* fused in-frame with a 497 amino acids sequence coding for the mature human GBA lacking its native 39 amino acids signal sequence (SEQ ID N°30) is used for the genetic transformation. The GBA protein contains 5 potential N-glycosylation sites.

a) Standard culture conditions of *Phaeodactylum tricornutum*
*Phaeodactylum tricornutum* strain used to express GBA is grown and prepared for genetic transformation as in example 1.a).
b) Expression constructs for genetic transformation
The vector used for the expression of human GBA is the same vector used for the expression of the chimeric protein EPO-GFP in example 1.b).
The sequence containing the bipartite topogenic signal sequence fused in-frame with the human GBA (nucleic acid sequence SEQ ID N°30) is synthesized with the addition of EcoRI and HindIII restriction sites flanking the 5' and 3' ends respectively. Alternatively, a similar sequence containing a histidine tag at the carboxy-terminal (GBA-HisTag) is also synthesized (nucleic acid sequence SEQ ID N°31). After digestion by EcoRI and HindIII, each insert is introduced into the pPHA-T1 vector. As a control, an empty pPha-T1 vector lacking the GBA coding sequence is used.
c) Genetic transformation
The genetic transformation carried out in this experiment is described in the previous example 1.c).
d) Microalgae DNA extraction
The DNA extraction carried out in this experiment is described in the previous example 1.d).
e) Polymerase chain reaction (PCR) analysis
The incorporation of the heterologous human GBA sequence in the genome of *Phaeodactylum tricornutum* is assessed by PCR analysis. The sequences of primers used for the PCR amplification are 5'-ATACCAAGCTCAAGATACC-3' (SEQ ID N°32) and 5'-AACTGTAACTTGTGCTCAGC-3' (SEQ ID N°33) located in the GBA coding sequence. The PCR reaction and agarose electrophoresis of PCR products are carried out as in example 1.e).
f) Immunoblotting analysis
Intracellular fractions of wild-type and transformed cells of *P. tricornutum* are prepared as previously described in example 1.g).
Ten μL of intracellular fractions from the various GBA expressing cells and wild-type cells are separated by SDS-PAGE using a 12% polyacrylamide gel. The separated proteins are transferred onto nitrocellulose membrane and stained with Ponceau Red in order to control transfer efficiency. Immunoblotting experiment is performed as described in example 1.g) except that the primary antibody is an anti-GBA (Santa Cruz, sc-100544) (1:1000 in TBS-T containing milk 1% for 2h at room temperature) and the secondary antibody is a horseradish peroxidase-conjugated bovine anti-mouse IgG (Santa Cruz, SC2371) (1:10000 in TBS-T containing milk 1% for 1.5h at room temperature).
Deglycosylation assay is performed on the various intracellular fractions as described previously in example 1.g) and analysed by immunoblotting experiment.
g) Purification of the β-glucocerebrosidase
β -glucocerebrosidase carrying the histidine tag (GBA-HisTag) is purified from intracellular fractions by chromatography method as described in example 1.h). Purified β-glucocerebrosidase is then analysed by immunoblotting experiment.
h) Structural characterization of N-linked glycans of the β-glucocerebrosidase
N-linked glycans are released from the β-glucocerebrosidase purified by affinity chromatography and analyzed by mass spectrometry as previously described in example 1.i).

**Example 3 Targeting of nuclear-encoded viral envelope glycoprotein into the plastid of *Phaeodactylum tricornutum***

The envelope spike of HIV contains various highly glycosylated proteins including gp120. Native N-linked glycans of gp120 are almost entirely oligomannose ($Man_{5-9}GlcNAc_2$) compared to the recombinant gp120 produced in the human cell line HEK293T which contains a majority of complex glycans. High-mannose glycans of gp120 ($Man_{6-9}GlcNAc_2$) are important determinant of antibodies recognition including 2G12, one of the most effective HIV neutralizing antibody. In the context of the viral vaccination design, the present invention thus confers a major advantage for the production of the envelope glycoprotein gp120 bearing high-mannose glycans, and used as antigens.
The viral envelope glycoprotein gp120 is expressed, targeted and stored into the plastidial stroma of *P. tricornutum* by means of the present invention. A plasmid containing a 55 amino acids bipartite topogenic signal sequence of the ATPase gamma subunit (atpC) from *P. tricornutum* fused in-frame with a 479 amino acids sequence coding for gp120 (SEQ ID N°34) is used for the genetic transformation. The envelope glycoprotein gp120 contains 24 putative N-glycosylation sites.

a) Standard culture conditions of *Phaeodactylum tricornutum*

*Phaeodactylum tricornutum* strain used to express gp120 is grown and prepared for genetic transformation as in example 1.a).

b) Expression constructs for genetic transformation

The vector used for the expression of gp120 is the same vector used for the expression of the chimeric protein EPO-GFP in example 1.b).

The sequence containing the bipartite topogenic signal sequence fused in-frame with gp120 coding sequence containing a stop codon (nucleic acid sequence SEQ ID N°34) is synthesized with the addition of EcoRI and HindIII restriction sites flanking the 5' and 3' ends respectively. Alternatively, a similar sequence containing a histidine tag at the carboxy-terminal (gp120-HisTag) (nucleic acid sequence SEQ ID N°35) is also synthesized with the addition of EcoRI and HindIII restriction sites flanking the 5' and 3' ends respectively. After digestion by EcoRI and HindIII, each insert is introduced into the pPHA-T1 vector. As a control, an empty pPha-T1 vector lacking the gp120 coding sequence is used.

c) Genetic transformation

The genetic transformation carried out in this experiment is described in the previous example 1.c).

d) Microalgae DNA extraction

The DNA extraction carried out in this experiment is described in the previous example 1.d).

e) Polymerase chain reaction (PCR) analysis

The incorporation of the heterologous viral gp120 sequence in the genome of *Phaeodactylum tricornutum* is assessed by PCR analysis. The sequences of primers used for the PCR amplification are 5'-CACCTCAGTCATTACACAGGC-3' (SEQ ID N°36) and 5'-CCTCCTGAGGATTGCTTAA-3' (SEQ ID N°37) located in the GP120 coding sequence respectively. The PCR reaction and agarose electrophoresis of PCR products are carried out as in example 1.e).

f) Immunoblotting analysis

Intracellular fractions of wild-type and transformed cells of *P. tricornutum* are prepared as previously described in example 1.g).

Ten μL of intracellular fractions from the various gp120 expressing cells and wild-type cells are separated by SDS-PAGE using a 12% polyacrylamide gel. The separated proteins are transferred onto nitrocellulose membrane and stained with Ponceau Red in order to control transfer efficiency. Immunoblotting experiment is performed as described in example 1.g) with a single anti-histidine tag antibody conjugated to horseradish peroxidase (Santa Cruz, sc-8036-HRP) (1:1000 in TBS-T containing milk 1% for 2h at room temperature).

Deglycosylation assay is performed on the various intracellular fractions as described previously in example 1.g) and analysed by immunoblotting experiment.

g) Purification of the glycoproteins gp120

The glycoprotein gp120 carrying the histidine tag is purified from intracellular fractions by chromatography method as described in example 1.h). Purified gp120 is then analysed by immunoblotting experiment.

h) Structural characterization of N-linked glycan of gp120

N-linked glycans are released from gp120 purified by affinity chromatography and analyzed by mass spectrometry as previously described in example 1.i).

## Claims

1. A transformed microalga producing a protein harboring a non-immunogenic "high mannose" pattern of glycosylation in the plastid of said transformed microalga, wherein

   1) said transformed microalga has a Chloroplast Endoplasmic Reticulum (CER);
   2) said microalga has been transformed with a nucleic acid sequence operatively linked to a promoter, said nucleic acid sequence encoding an amino acid sequence comprising:

      *(i)* An amino-terminal bipartite topogenic signal (BTS) sequence composed of at least a signal peptide followed by a transit peptide; and
      *(ii)* The sequence of said protein;

   3) the xylosyltransferases and fucosyltransferases of said microalga have not been inactivated;
   4) The N-acetylglycosyltransferase I of said microalga has not been inactivated, preferably the N-acetylglycosyltranferases II, III, IV, V and VI, mannosidase II and glycosyltransferases of said microalga have not been inactivated.

2. The transformed microalga according to claim 1 wherein said microalga having a CER is selected from the group comprising heterokonts, cryptophytes and haptophytes microalgae.

3. The transformed microalga according to any one of claims 1 or 2 wherein said microalga having a CER is selected from the group comprising *Phaeodactylum, Nannochloropsis, Nitzschia, Skeletonema, Chaetoceros, Odontella, Amphiprora, Thalassiosira, Emiliania, Pavlova, Isochrysis, Apistonema* and *Rhodomonas.*

4. The transformed microalga according to any one of claims 1 to 3 wherein said microalga is the diatom *Phaeodactylum tricornutum.*

5. The transformed microalga according to any one of claims 1 to 4, wherein said BTS sequence leads to the translocation of the nucleic acid sequence encoding the BTS sequence and the sequence of a protein in the Endoplasmic Reticulum further followed by the transport of said protein in the plastid of the microalga.

6. The transformed microalga according any one of claims 1 to 5 wherein said protein is a heterologous protein.

7. The transformed microalga according to any one of claims 1 to 6 wherein said protein present a pattern of glycosylation with at least one exposed mannose residue and between five to nine mannose residues, preferably from six to nine mannose residues, on the oligosaccharides located at the level of the asparagine residues of the consensus sequences Asn-X-Ser/Thr, when X is different than proline and aspartic acid, of said protein.

8. The transformed microalga according any one of claims 1 to 9, wherein said protein is selected in the group comprising lysosomal enzymes, viral envelope glycoproteins, antibodies or antibodies' fragments and derivatives thereof.

9. The transformed microalga according any one of claims to 1 to 10, wherein said amino acid sequence encoding said protein is selected from the group comprising the amino acid sequences as listed in the following table and derivatives thereof :

| PROTEIN | CDS SEQ ID N° | Accession number (Protein) | Comments |
|---|---|---|---|
| β-glucocerebrosidase = Acid β-glucosidase | SEQ ID N° 7 | AAA35873 | Lysosomal enzyme |
| α-Galactosidase A | SEQ ID N°8 | NP_000160 | Lysosomal enzyme |
| Alglucosidase = Acid α-glucosidase | SEQ ID N°9 | NP_000143 | Lysosomal enzyme |
| α-L-iduronidase | SEQ ID N°10 | NP_000194 | Lysosomal enzyme |
| Iduronate 2-sulfatase | SEQ ID N°11 | NP_000193 | Lysosomal enzyme |
| Arylsulfatase B | SEQ ID N°12 | NP_000037 | Lysosomal enzyme |
| Acid Sphingomyelinase | SEQ ID N°13 | NP_000534 | Lysosomal enzyme |
| Lysosomal acid lipase | SEQ ID N°14 | NP_001121077 | Lysosomal enzyme |
| GP120 | SEQ ID N°15 | NP_579894 | Envelope glycoprotein from Human Immunodeficiency Virus 1 |
| GP41 | SEQ ID N°16 | NP_579895 | Envelope transmembrane glycoprotein from Human Immunodeficiency Virus 1 |
| E1 protein | SEQ ID N°17 | From aa 192 to 383 of the polyprotein P27958 | Envelope glycoprotein from Hepatitis C Virus |
| E2 protein | SEQ ID N°18 | From aa 384 to 746 of the polyprotein P27958 | Envelope glycoprotein from Hepatitis C Virus |
| E protein | SEQ ID N°19 | From aa 281 to 775 of the polyprotein ADO97105 | Envelope glycoprotein from Dengue virus 1 |
| E protein | SEQ ID N°20 | From aa 291 to 791 of the polyprotein ADL27981 | Envelope glycoprotein from West Nile Virus |
| Spike glycoprotein precursor | SEQ ID N°21 | ACI28632 | Envelope glycoprotein from Ebola virus |
| immunoglobulin heavy chain constant region gamma | SEQ ID N°22 | CAC20454 | Gamma 1 |
| | SEQ ID N°23 | CAC20457 | Gamma 4 |
| Immunoglobulin Variable Heavy Chain | SEQ ID N°24 | AAA59127 | |
| Immunoglobulin Kappa light Chain (VL+CL) | SEQ ID N°25 | CAA09181 | |

**10.** A protein harboring a high mannose pattern of glycosylation produced by the method according to any one of claims 12 to 13.

**11.** A composition comprising a protein according to claim 10.

**12.** A method for producing a protein harboring a "high mannose" pattern of glycosylation in the plastid of a transformed microalga having a Chloroplastic Endoplasmic Reticulum (CER) as claimed in any one of claims 1 to 10, said method comprising the steps of:

1) Culturing said transformed microalga;
2) Harvesting the plastid of said transformed microalga;
3) Purifying said protein from said plastid.

**13.** The method according to claim 12 wherein said method comprises a step 4) of determining the glycosylation pattern of said protein and conserving the protein harboring a high mannose pattern of glycosylation.

**14.** The use of a transformed microalga having a CER as defined in any one of claims 1 to 9 for the production of a polypeptide harboring a non-immunogenic "high mannose" pattern of glycosylation in the plastid of said microalga.

Europäisches Patentamt
European Patent Office
Office européen des brevets

# EUROPEAN SEARCH REPORT

Application Number
EP 10 01 6162

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br>Y | EP 2 090 648 A1 (IFREMER [FR]; CENTRE NAT RECH SCIENT [FR]; UNIV ROUEN [FR])<br>19 August 2009 (2009-08-19)<br>* the whole document *<br>----- | 10,11<br><br><br>1-9,<br>12-14 | INV.<br>C12N15/82<br>C12P21/00<br><br>ADD.<br>C12R1/89 |
| Y | GOULD SVEN B: "Ariadne's thread: Guiding a protein across five membranes in cryptophytes",<br>JOURNAL OF PHYCOLOGY,<br>vol. 44, no. 1, February 2008 (2008-02), pages 23-26, XP002639507,<br>ISSN: 0022-3646<br>* the whole document *<br>----- | 1-9,<br>12-14 | |
| Y | ANSGAR GRUBER ET AL: "Protein targeting into complex diatom plastids: functional characterisation of a specific targeting motif",<br>PLANT MOLECULAR BIOLOGY, KLUWER ACADEMIC PUBLISHERS, DORDRECHT, NL,<br>vol. 64, no. 5, 5 May 2007 (2007-05-05), pages 519-530, XP019507439,<br>ISSN: 1573-5028, DOI:<br>DOI:10.1007/S11103-007-9171-X<br>* the whole document *<br>----- | 1-9,<br>12-14 | TECHNICAL FIELDS SEARCHED (IPC)<br><br>C12N<br>C12P |
| Y | APT KIRK E ET AL: "In vivo characterization of diatom multipartite plastid targeting signals",<br>JOURNAL OF CELL SCIENCE,<br>vol. 115, no. 21,<br>1 November 2002 (2002-11-01), pages 4061-4069, XP002639508,<br>ISSN: 0021-9533<br>* the whole document *<br>-----<br>-/-- | 1-9,<br>12-14 | |

The present search report has been drawn up for all claims

1

| Place of search | Date of completion of the search | Examiner |
|---|---|---|
| The Hague | 30 May 2011 | Kools, Patrick |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 01 6162

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | LEON-BANARES R ET AL: "Transgenic microalgae as green cell-factories", TRENDS IN BIOTECHNOLOGY, ELSEVIER PUBLICATIONS, CAMBRIDGE, GB, vol. 22, no. 1, 1 January 2004 (2004-01-01), pages 45-52, XP004481957, ISSN: 0167-7799, DOI: DOI:10.1016/J.TIBTECH.2003.11.003 * the whole document * ----- | 1-14 | |
| | | | TECHNICAL FIELDS SEARCHED (IPC) |

The present search report has been drawn up for all claims

1

| Place of search | Date of completion of the search | Examiner |
|---|---|---|
| The Hague | 30 May 2011 | Kools, Patrick |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 01 6162

This annex lists the patent family members relating to the patent documents cited in the above-mentioned European search report.
The members are as contained in the European Patent Office EDP file on
The European Patent Office is in no way liable for these particulars which are merely given for the purpose of information.

30-05-2011

| Patent document cited in search report | Publication date | Patent family member(s) | Publication date |
|---|---|---|---|
| EP 2090648 A1 | 19-08-2009 | AU 2009214077 A1 | 20-08-2009 |
| | | CA 2715107 A1 | 20-08-2009 |
| | | EP 2257618 A1 | 08-12-2010 |
| | | WO 2009101160 A1 | 20-08-2009 |
| | | US 2011045533 A1 | 24-02-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BOLTE et al.** Protein targeting into secondary plastids. *Journal of Eukaryotic Microbiology,* 2009, 9-15
- **APT et al.** In vivo characterization of diatom multipartite plastid targeting signals. *Journal of Cell Science,* 2002, 4061-4069
- **VON HEIJNE.** The signal Peptide. *Journal of Membrane Biology,* 1990, 195-201
- **EMANUELSSON et al.** Locating proteins in the cell using TargetP, SignalP and related tools. *Nature Protocols,* 2007, 953-971
- **VERNER ; SCHATZ.** Protein translocation across membranes. *Science,* 1988, 1307-1313
- **JARVIS.** Targeting of nucleus-encoded proteins to chloroplasts in plants. *New Phytologist,* 2008, 257-285
- **DOLASHKA et al.** Glycan structures and antiviral effect of the structural subunit RvH2 of Rapana hemocyanin. *Carbohydrate Research,* 2010, 2361-2367
- **SÉVENO et al.** 2008) Plant N-glycan profiling of minute amounts of material. *Analytical Biochemistry,* 2008, 66-72
- **ZASLAVSKAIA ; LIPPMEIER.** transformation of the diatom Phaeodactylum tricornutum (Bacillariophyceae) with a variety of selectable marker and reporter genes. *Journal of Phycology,* 2000, 379-386
- **THOMAS et al.** A helium burst biolistic device adapted to penetrate fragile insect tissues. *Jounal of Insect Science,* 2001, 1-9
- **DOLASHKA et al.** Glycan structures and antiviral effect of the structural subunit RvH2 of Rapana hemocyanin. *Carbohydr Res,* 2010, vol. 345, 2361-2367